# EUROPEAN PATENT SPECIFICATION

(11) **EP 4 328 227 B1**
(45) Date of publication and mention of the grant of the patent: **19.08.2026**
(21) Application number: 22791100.5
(22) Date of filing: 21.04.2022
(51) Int. Cl.: C07D 487/04, A61K 31/53, A61K 31/5377, A61P 31/14, A61P 29/00

(54) **NUCLEOSIDE COMPOUND AND APPLICATION THEREOF IN THE TREATMENT OF FELINE INFECTIOUS PERITONITIS**
NUKLEOSIDVERBINDUNG UND ANWENDUNG DAVON BEI DER BEHANDLUNG VON INFEKTIÖSER PERITONITIS BEI KATZEN
COMPOSÉ NUCLÉOSIDIQUE ET SON UTILISATION DANS LE TRAITEMENT DE LA PÉRITONITE INFECTIEUSE FÉLINE

(30) Priority: 23.04.2021 CN 202110442975
(43) Date of publication of application: 28.02.2024
(73) Proprietor: Vigonvita Life Sciences Co., Ltd., Suzhou, Jiangsu 215123 (CN)
(72) Inventor: LEI, Yang, Suzhou, Jiangsu 215123 (CN); CAO, Gang, Suzhou, Jiangsu 215123 (CN); HU, Rong, Suzhou, Jiangsu 215123 (CN)
(74) Representative: karo IP
(86) International application number: PCT/CN2022/088179
(87) International publication number: WO 2022/222994

(56) References cited:
- EP-A1- 4 141 007
- WO-A1-2018/169946
- WO-A1-2022/142477
- WO-A2-2022/047065
- CN-A- 111 135 184
- CN-A- 111 961 057
- CN-A- 111 961 057
- CN-A- 112 778 310
- CN-A- 113 185 519
- CN-A- 113 735 862
- MACKMAN RICHARD L. ET AL: "Prodrugs of a 1'-CN-4-Aza-7,9-dideazaadenosine C -Nucleoside Leading to the Discovery of Remdesivir (GS-5734) as a Potent Inhibitor of Respiratory Syncytial Virus with Efficacy in the African Green Monkey Model of RSV", JOURNAL OF MEDICINAL CHEMISTRY, vol. 64, no. 8, 9 April 2021 (2021-04-09), US, pages 5001 - 5017, XP055926529, ISSN: 0022-2623, DOI: 10.1021/acs.jmedchem.1c00071
- LI JIAPENG ET AL: "Tissue-Specific Proteomics Analysis of Anti-COVID-19 Nucleoside and Nucleotide Prodrug-Activating Enzymes Provides Insights into the Optimization of Prodrug Design and Pharmacotherapy Strategy", ACS PHARMACOLOGY & TRANSLATIONAL SCIENCE, vol. 4, no. 2, 1 April 2021 (2021-04-01), pages 870 - 887, XP055948009, ISSN: 2575-9108, DOI: 10.1021/acsptsci.1c00016
- "Molecular Pharmaceutics", 31 January 2010, HUNAN NORMAL UNIVERSITY PRESS, CN, ISBN: 7-5648-0114-X, article CHEN, YUXIANG: "Passage; Molecular Pharmaceutics", pages: 197, XP009540903

## Description

### RELATED APPLICATIONS

The present disclosure claims priority to Chinese Patent Application No. CN202110442975.X, entitled "Nucleoside compound and application thereof in the treatment of feline infectious peritonitis", filed on April 23, 2021.

### TECHNICAL FIELD

The present disclosure relates to a nucleoside compound and an application thereof in the treatment of feline infectious peritonitis.

### BACKGROUND

Feline infectious peritonitis (FIP) is a chronic, progressive, and fatal infectious disease caused by infection with feline coronavirus. The disease is commonly seen in two forms clinically. One is the exudative type characterized by peritonitis and large amounts of ascites, also known as wet-type infectious peritonitis. One is dry infectious peritonitis characterized by uveitis such as fibrin blood clots and retinal hemorrhage in the eyes without obvious symptom changes in the whole body.

At present, the treatment of feline infectious peritonitis mainly uses immunomodulators, immunosuppressants, antiviral, and other drugs for symptomatic treatment. These drugs have significant side effects and cannot reduce the high mortality rate of the disease. Therefore, there is an urgent need to develop efficient and safe drugs.

Feline coronavirus (FCoV) is an RNA virus and a common infectious virus in Felidae. Cats infected with feline coronavirus have a 5-10% probability of mutating into feline infectious peritonitis (FIP). The main symptoms are peritonitis and large amounts of pleural fluid, and the mortality rate of sick cats is extremely high. Currently, GS-441524 is a drug molecule that is effective in treating feline infectious peritonitis (FIP).

GS-441524 is 1'-cyano-substituted adenine C-nucleoside ribose analogue that exhibits strong antiviral activity against various RNA viruses, including SARS coronavirus, and has effective inhibitory effects against various RNA viruses. Among them, GS-441524 is phosphorylated into an active triphosphate metabolite (NTP structural analog) by cellular kinases in cells. In viral RNA synthesis, the active NTP structural analog acts as a competitor of natural triphosphate nucleosides and competes with natural nucleosides (NTP) to participate in RNA transcription/replication. When the GS-441524 molecule is inserted into the transcription/replication product, transcription/replication will be terminated prematurely, thereby inhibiting the viral RNA transcription/replication process. At present, it has been found that it exhibits certain inhibitory activity against Ebola, SARS, Junin virus, respiratory syncytial virus, etc.

Document WO2018/169946A1 discloses methods and compounds for treating feline Coronavirus infections, particularly methods for treating feline infectious peritonitis virus (FIPV).

Document CN111961057A discloses an α-configuration nucleoside and a corresponding prodrug, a solvate thereof, or a pharmaceutically acceptable salt, a pharmaceutical composition comprising the compound, and a method for treating cats or other animals Application for coronavirus infection.

Document "Prodrugs of a 1'-CN-4-Aza-7,9-dideazaadenosine C-Nucleoside Leading to the Discovery of Remdesivir (GS-5734) as a Potent Inhibitor of Respiratory Syncytial Virus with Efficacy in the African Green Monkey Model of RSV" details the discovery and development of remdesivir (GS-5734), a potent antiviral drug initially identified as a treatment for respiratory syncytial virus (RSV) through phenotypic screening.

Document WO2022/142477A1 discloses methods and compounds for treating coronavirus infections, particularly methods and nucleosides for treating SARS-CoV-2 infections or infections caused by SARS-CoV-2 variants.

Document WO2022/047065A2 discloses compounds and methods for treatment of viral infections.

Document EP4141007A1 discloses an antiviral application of nucleoside analog or combination formulation containing nucleoside analog.

### SUMMARY

The water solubility of the GS441524 molecule is poor, and its membrane permeability and bioavailability are also relatively limited. Therefore, the novel nucleoside compounds reported in the present disclosure have good water solubility and antiviral effects, especially against feline coronavirus. The invention is set out in the appended set of claims.

The present disclosure provides a compound with a structure as shown in formula (I) or a pharmaceutically acceptable salt thereof: 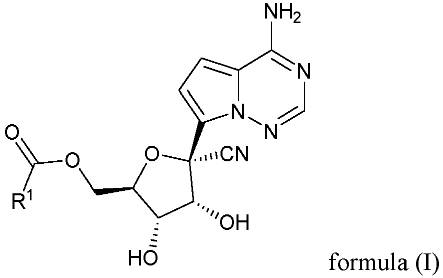 wherein,
the compound has one of the following structures:

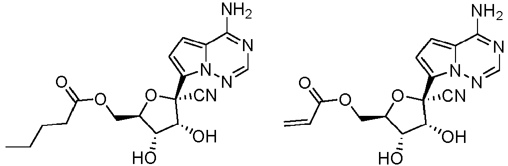

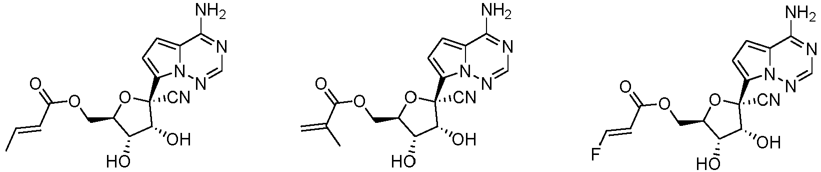

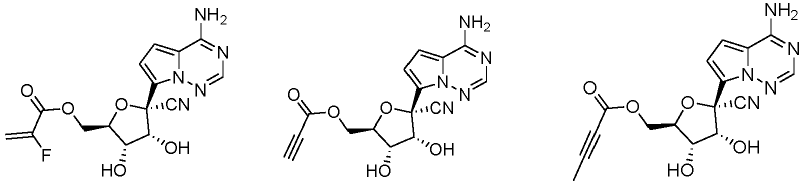

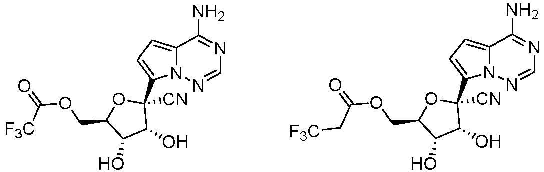

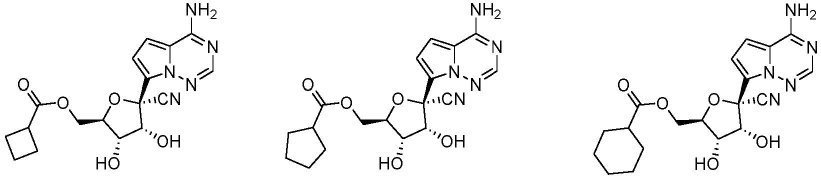

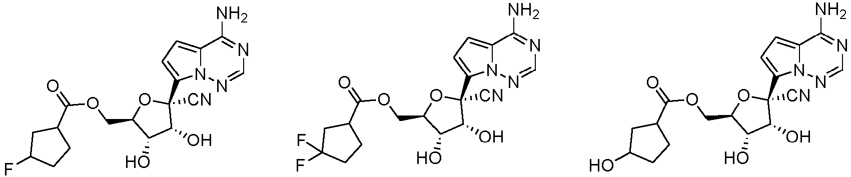

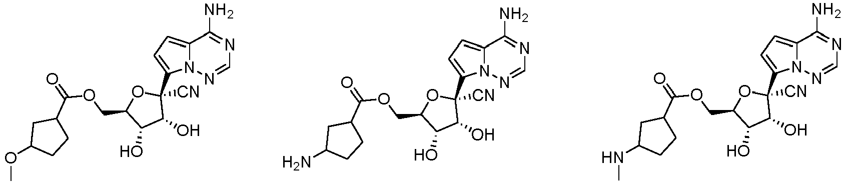

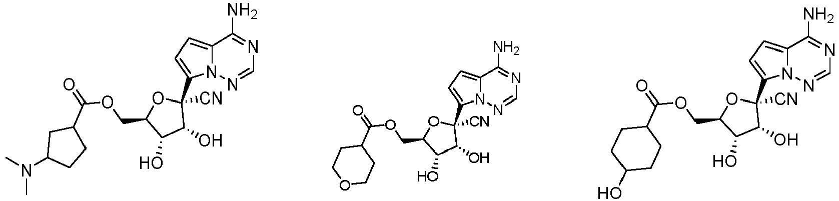

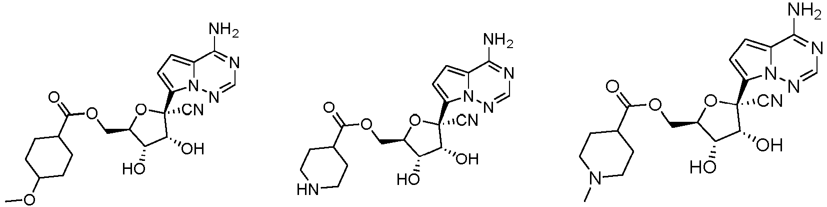

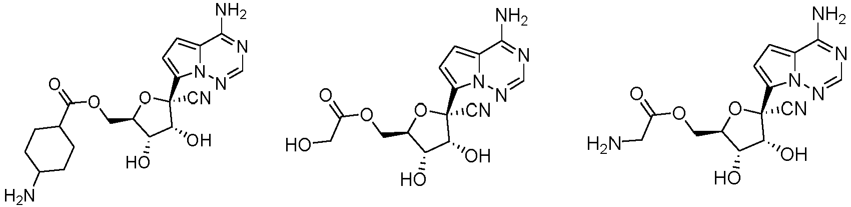

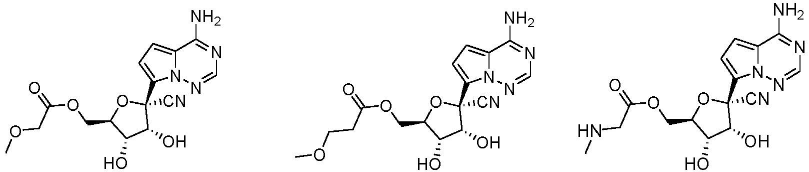

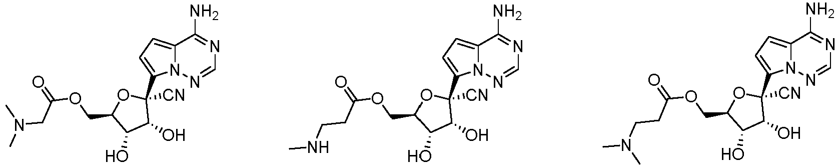

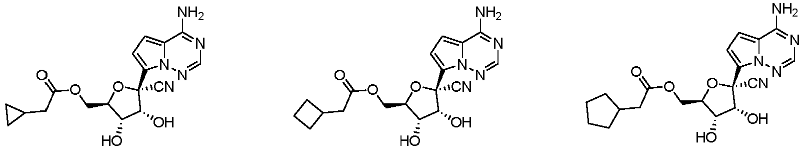

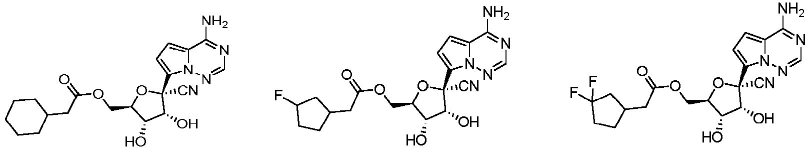

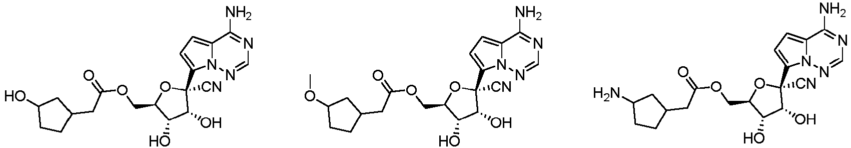

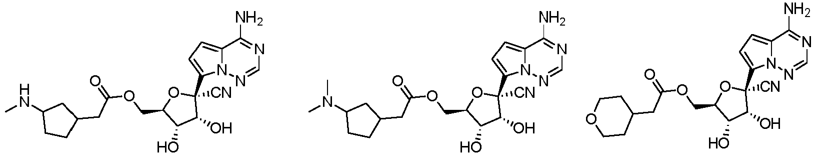

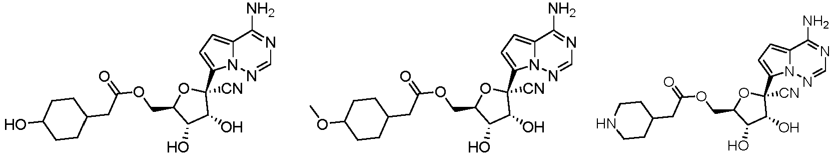

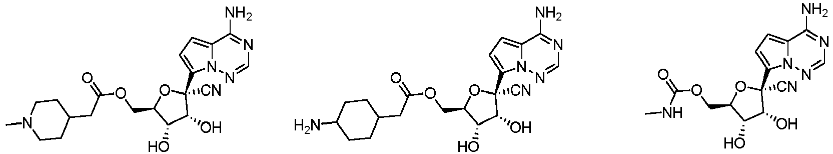

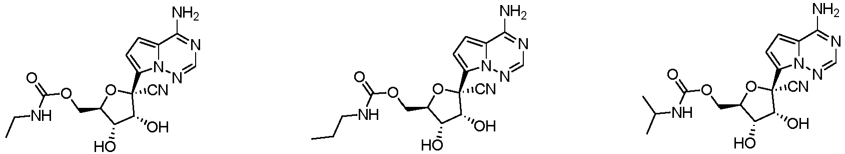

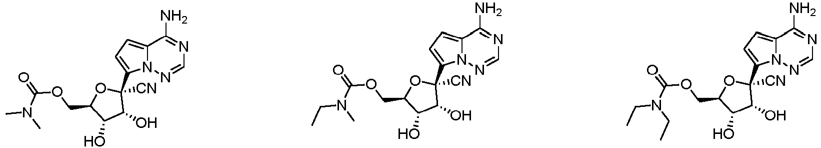

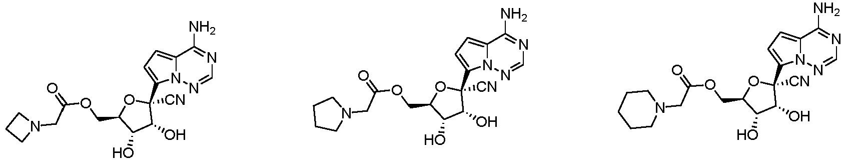

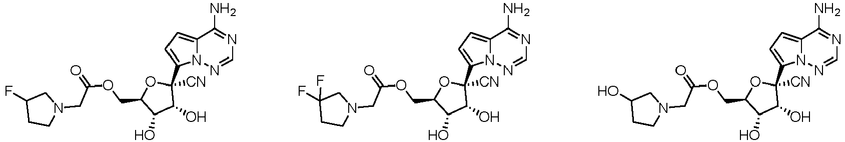

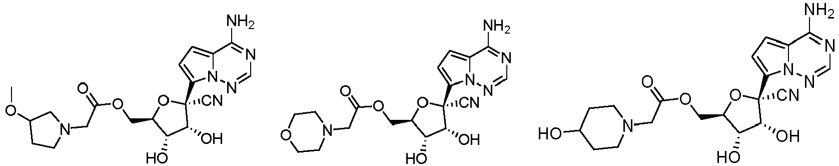

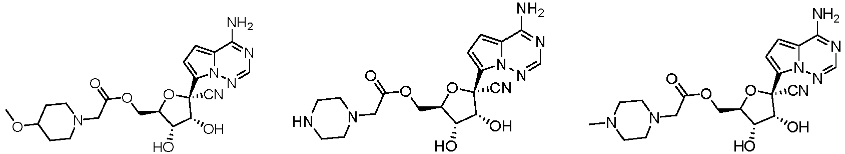

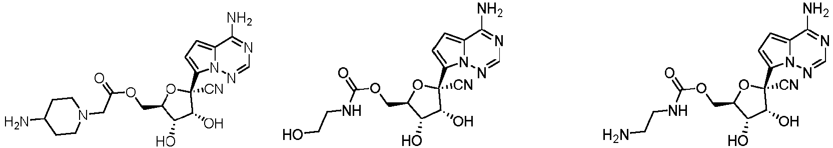

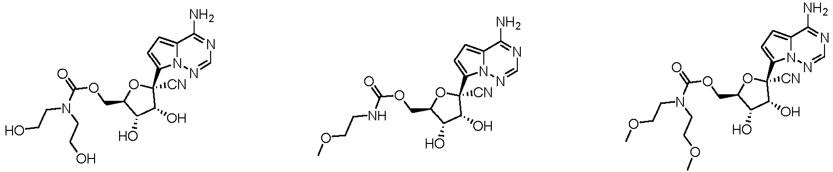

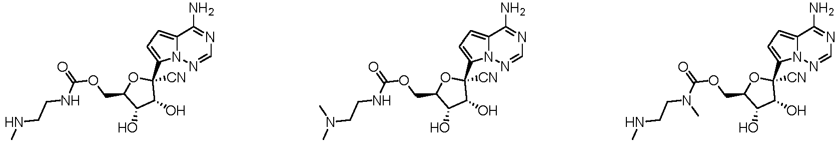

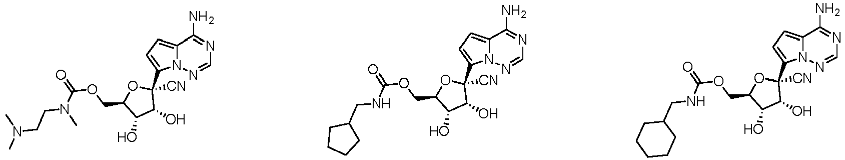

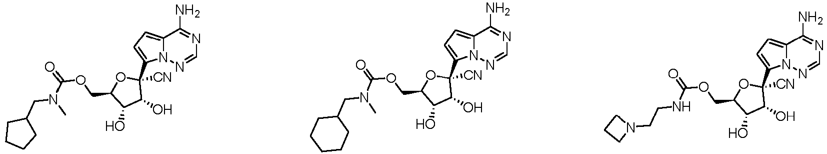

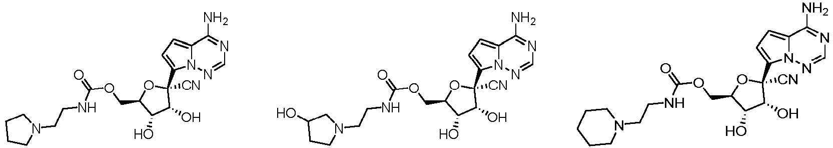

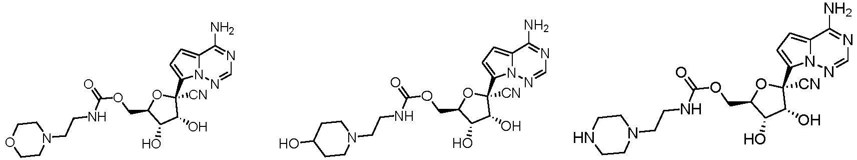

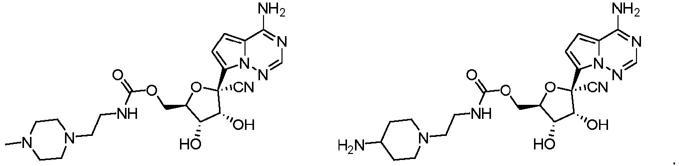

In some embodiments, the disclosure provides a pharmaceutical composition, which contains pharmaceutically acceptable excipients or carriers and the compound described in formula (I) of the present disclosure or a pharmaceutically acceptable salt thereof, including each optical isomer, each crystal form, hydrate, or solvate as active ingredients.

In some embodiments, the disclosure provides a use of the compound of the present disclosure, or a pharmaceutically acceptable salt thereof in the treatment of diseases caused by feline coronavirus infection, namely feline infectious peritonitis.

It should be understood that the foregoing general description and the following detailed description of the present disclosure are both exemplary and explanatory, intending to provide further explanation of the claimed disclosure.

### BRIEF DESCRIPTION OF DRAWINGS

The present disclosure will be further described below in conjunction with the accompanying drawings and examples.
Figure 1 shows the weight changes of the affected cat in two weeks before and after treatment with aqueous injection of the compound of the present disclosure;
Figure 2 shows the changes in the number of white blood cells in the affected cat in two weeks before and after treatment with aqueous injection of the compound of the present disclosure;
Figure 3 shows the changes in the ratio of Albumin to Globulin (A/G) of the affected cat in two weeks before and after treatment with aqueous injection of the compound of the present disclosure.

### DETAILED DESCRIPTION

Certain embodiments of the disclosure are now described in detail, examples of which are illustrated by the accompanying structural and chemical formulas. The present disclosure intends to cover all alternatives, modifications, and equivalent technical solutions, all of which are included within the scope of the present disclosure as defined in the claims. Those skilled in this art should recognize that many methods and materials similar or equivalent to those described herein can be used to practice the present disclosure. The present disclosure is not limited to the methods and materials described herein. In the event that one or more of the literature, patents, and similar materials combined differ from or conflict with this application (including but not limited to the defined terms, application of terms, described technology, etc.), this application shall prevail.

Unless otherwise specified, the following definitions as used herein should apply. For the purpose of the present disclosure, chemical elements are defined according to the Periodic Table of Chemical Elements, CAS Edition, and Handbook of Chemicals, 75th Edition, 1994. In addition, the general principles of organic chemistry can be referred to in "Organic Chemistry," by Thomas Sorrell, University Science Books, Sausalito: 1999, and "March's Advanced Organic Chemistry", by Michael B. Smith and Jerry March, John Wiley & Sons, New York: 2007.

"Pharmaceutically acceptable salts" of the present disclosure refer to organic salts and inorganic salts of the compounds of the present disclosure. Pharmaceutically acceptable salts are well known in the art. For example, S. M. Berge, et al., describes pharmaceutically acceptable salts in detail in J Pharmaceutical Sciences, 66: 1-19 (1977). Examples of pharmaceutically acceptable salts formed by non-toxic acids include but are not limited to: inorganic acid salts formed with amino groups by reaction such as hydrochloride, hydrobromide, phosphate, sulphate, and perchlorate, or organic acid salts such as acetate, oxalate, maleate, tartrate, citrate, succinate, and malonate, or these salts can be obtained by other methods described in books and literature, such as ion exchange method. Other pharmaceutically acceptable salts include adipate, malate, 2-hydroxy propionate, alginate, ascorbate, aspartate, benzenesulfonate, benzoate, bisulfate, borate, butyrate, camphorate, camphor sulfonate, cyclopentyl propionate, digluconate, lauryl sulfate, ethanesulfonate, formate, fumarate, glucoheptanoate, glycerophosphate, gluconate, hemisulfate, enanthate, caproate, hydroiodide, 2-hydroxyethanesulfonate, lacturonate, lactate, laurate, lauryl sulfate, malate, malonate, methanesulfonate, 2-naphthalenesulfonate, nicotinate, nitrate, oleate, palmitate, pamoate, pectate, persulfate, 3-phenylpropionate, picrate, pivalate, propionate, stearate, thiocyanate, p-toluenesulfonate, undecanoate, valerate, and the like. Salts obtained with appropriate bases include alkali metal, alkaline earth metal, ammonium, and N⁺(C1-4 alkyl)₄ salts. The present disclosure also contemplates the formation of quaternary ammonium salts of any compound containing an N group. Water-soluble or oil-soluble or dispersed products can be obtained by quaternization. Alkali or alkaline earth metals that can form salts include sodium, lithium, potassium, calcium, magnesium, and the like. Pharmaceutically acceptable salts further include appropriate, non-toxic ammonium, quaternary ammonium salts, and counter ion forming amine cations, such as halides, hydroxides, carboxylates, sulfates, phosphates, nitrates, C1-S sulfonates, and aromatic sulfonates.

"Solvate" as used herein refers to an association of one or more solvent molecules with a compound of the present disclosure. Solvents that form solvates include, but are not limited to: water, isopropyl alcohol, ethanol, methanol, dimethyl sulfoxide, ethyl acetate, acetic acid, and aminoethanol. The term "hydrate" refers to an association of solvent molecules with water.

In addition, the compounds disclosed in the present disclosure, including their salts, can also be obtained in the form of their hydrates or in the form of solvents (such as ethanol, DMSO, etc.) used for their crystallization. The compounds disclosed herein may form solvates, intrinsically or by design, with pharmaceutically acceptable solvents, including water; thus, the present disclosure is intended to encompass both solvated and unsolvated forms.

The compounds disclosed herein, including their salts, can be prepared in various forms, including, but not limited to, amorphous, pulverized, and nano-particulate forms. In addition, the compounds of the present disclosure include crystalline forms and may also be polymorphic forms. Polymorphs include different lattice arrangements of the same elemental composition of a compound. Polymorphs usually have different X-ray diffraction patterns, infrared spectra, melting points, density, hardness, crystalline form, optical and electrical properties, stability, and solubility. Different factors such as recrystallization solvent, crystallization rate, and storage temperature may cause a single crystalline form to dominate.

As described in the present disclosure, 'pharmaceutically acceptable excipients or carriers', including any solvent, diluent, or other liquid excipients, dispersing or suspending agent, surfactant, isotonic agent, thickener, emulsifier, preservative, solid binder or lubricant, etc., are suitable for the specific target dosage form. As described in the following literature: In Remington: The Science and Practice of Pharmacy, 21st edition, 2005, ed. D. B. Troy, Lippincott Williams & Wilkins, Philadelphia, and Encyclopedia of Pharmaceutical Technology, eds. J. Swarbrick and J. C. Boylan, 1988-1999, Marcel Dekker, New York. A review of the literature herein demonstrates that various carriers may be used in the formulation of pharmaceutically acceptable compositions and the well-known methods for their preparation. In addition to the scope of incompatibility between any conventional carrier medium and the compounds of the present disclosure, such as any adverse biological effects or harmful interactions with any other component of a pharmaceutically acceptable composition, their use is also considered within the scope of the present disclosure.

Substances that can be used as pharmaceutically acceptable excipients or carriers include, but are not limited to: ion exchangers, aluminum, aluminum oxide, aluminum stearate, lecithin, serum proteins such as human serum albumin, buffering substances such as phosphate, glycine, sorbic acid, potassium sorbate, partial glyceride mixtures of saturated plant fatty acids, water, electrolytes, such as sulfated protamine, disodium hydrogen phosphate, potassium hydrogen phosphate, salts, such as sodium chloride, zinc salt, colloidal silicon, magnesium trisilicate, polyvinylpyrrolidone, polyacrylate, wax, polyethylene-polyoxypropylene-blocking polymers, lanolin, sugars, such as lactose, glucose, and sucrose; starch such as corn starch and potato starch; cellulose and its derivatives such as sodium carboxymethyl cellulose, ethyl cellulose, and cellulose acetate; gum powder; malt; gelatin; talcum powder; excipients such as cocoa butter and suppository wax; oils such as peanut oil, cottonseed oil, safflower oil, sesame oil, olive oil, corn oil, and soybean oil; glycol compounds, such as propylene glycol and polyethylene glycol; esters such as ethyl oleate and ethyl laurate; agar; buffers such as magnesium hydroxide and aluminum hydroxide; alginic acid; pyrogen-free water; isotonic salt; Ringer's solution; ethanol; phosphate buffer solution; and other non-toxic suitable lubricants such as sodium laurate and magnesium stearate, coloring agents, release agents, coating materials, sweeteners, flavors and fragrances, preservatives and antioxidants. For convenience, local anesthetics, preservatives, buffers, and the like can be directly dissolved in the vehicle.

The term "alkyl" used in the present disclosure refers to a saturated straight-chain or branched monovalent hydrocarbon group of 1 to 20 carbon atoms, wherein the alkyl group can be independently and optionally substituted by one or more substituents described in the present disclosure. In some embodiments, the alkyl group contains 1-10 carbon atoms. In other embodiments, the alkyl group contains 1-8 carbon atoms. In other embodiments, the alkyl group contains 1-6 carbon atoms. In other embodiments, the alkyl group contains 1-4 carbon atoms. In other embodiments, the alkyl group contains 1-3 carbon atoms. Further examples of alkyl groups include, but are not limited to, methyl (Me, -CH₃), ethyl (Et, -CH₂CH₃), n-propyl (n-Pr, -CH₂CH₂CH₃), isopropyl (i-Pr, -CH (CH3)₂), n-butyl (-Bu, -CH₂CH₂CH₂CH₃), 2-methylpropyl or isobutyl (i-Bu, -CH2CH(CH3) ₂), 1-methylpropyl base or sec-butyl (s-Bu, -CH(CH₃)CH₂CH₃), tert-butyl (t-Bu, -C(CH₃)₃), n-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl ( - CH₂CH₂CH(CH₃)₂), 2-methyl-1-butyl (-CH₂CH(CH₃)CH₂CH₃), n-hexyl (-CH₂CH₂CH₂CH₂CH₂CH₃), 2-hexyl(-CH(CH₃)CH₂CH₂CH₂CH₃), 3-hexyl(-CH(CH₂CH₃)(CH₂CH₂CH₃)), 2-methyl-2-pentyl(-C(CH₃) ₂CH₂CH₂CH₃), 3-methyl-2-pentyl(-CH(CH₃)CH(CH₃)CH₂CH₃), 4-methyl-2-pentyl(-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃) ₂CH(CH₃) ₂), 3,3-dimethyl-2-butyl (-CH(CH₃)C(CH₃)₃), n-heptyl, n-octyl, etc. The term "alkyl" and its prefix "alkyl" as used herein include both straight and branched saturated carbon chains.

The term "alkenyl" used in the present disclosure refers to unsaturated aliphatic hydrocarbon groups containing carbon-carbon double bonds, including linear or branched chain groups with 1 to 6 carbon atoms. Preferably, lower alkenyl groups containing 1 to 4 carbon atoms, such as vinyl, 1-propenyl, 1-butenyl, or 2-methylpropenyl.

The term "alkynyl" used in the present disclosure refers to a monovalent hydrocarbon group with 2-12 carbon atoms in a linear or branched chain, in which at least one position is unsaturated, that is, one C-C is a sp triple bond. Wherein, the alkynyl group can be independently and optionally substituted by one or more substituents described in the present disclosure. Specific examples include but are not limited to, ethynyl ( 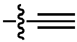 ), propargyl ( 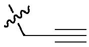 ), and the like.

The term 'alkoxy' used in the present disclosure relates to alkyl groups, as defined in the present disclosure, attached to the primary carbon chain through oxygen atoms. Unless otherwise specified, such alkoxy groups contain 1-12 carbon atoms. In one embodiment, the alkoxy group contains 1-6 carbon atoms. In another embodiment, the alkoxy group contains 1-4 carbon atoms. In another embodiment, the alkoxy group contains 1-3 carbon atoms. Such embodiments include but are not limited to, methoxy, ethoxy, propoxy, and the like.

The terms "haloalkyl" or "haloalkoxy" used in the present disclosure mean that alkyl or alkoxy groups can be substituted by one or more identical or different halogen atoms. Among them, alkyl and alkoxy groups have the meanings described in the present disclosure. Such examples include, but are not limited to, 1,1,1-trifluoro-2-methylpropan-2-yl (-C(CH₃)₂CF₃), 1,1-difluoro-2-methylpropan-2-yl (-C(CH₃)₂CHF₂), 1-fluoro-2-methylpropan-2-yl (-C(CH₃)₂CH₂F), difluoromethyl (-CH₂), trifluoromethyl (-CF₃), trifluoromethoxy (-OCF₃), 2,2,2-trifluoroethoxy (-OCH₂CF₃), 2,2,3,3-tetrafluoropropyloxy (-OCH₂CF₂CHF₂), etc.

The terms "carbocyclic" or "cyclic aliphatic", "carbocyclic", and "cycloalkyl" used in the present disclosure refer to monovalent or polyvalent, non-aromatic, saturated or partially unsaturated rings, and do not contain heteroatoms, including monocyclic rings with 3-12 carbon atoms or bicyclic or tricyclic rings with 7-12 carbon atoms. A bicyclic carbocyclic ring with 7-12 atoms can be a bicyclic [4,5], [5,5], [5,6] or [6,6] system, while a bicyclic carbocyclic ring with 9 or 10 atoms can be a bicyclic [5,6] or [6,6] system. Depending on the structure, "carbocyclic group" or "cyclic aliphatic group", "carbocyclic ", and "cycloalkyl group" can be monovalent or divalent groups, that is, in some embodiments of the present disclosure, they can be used instead of or as a carbocyclylene group or a cycloalkylene group. Suitable cyclic aliphatic groups include but are not limited to, cycloalkyl, cycloalkenyl, and cycloalkynyl groups. Examples of cyclic aliphatic groups further include, but are not limited to, cyclopropyl, cyclobutyl, cyclopentyl, 1-cyclopentyl-1-enyl, 1-cyclopentyl-2-enyl, 1-cyclopentyl-3-enyl, cyclohexyl, 1-cyclohexyl-1-enyl, 1-cyclohexyl-2-enyl, 1-cyclohexyl-3-enyl, cyclohexadienyl, cycloheptyl, cyclooctyl, cyclononyl, cyclodecyl, cycloundecyl, cyclododecyl, adamantyl, etc. And the "carbocyclyl" or "cyclic aliphatic group", " carbocycle", and "cycloalkyl " can be substituted or unsubstituted, wherein the substituent can be, but is not limited to, oxo (=O), fluorine, chlorine, bromine, iodine, hydroxyl, amino, -C(=O)-NH₂, carboxyl, -S(=O)tO-H, -OS(=O)t-H, -S(=O)ₜNH₂, triazolyl, tetrazolyl, -(CR^{3b}R^{3c})n-NH₂, alkyl, alkyl-S(=O)t-, haloalkyl, hydroxyalkyl, alkoxy, alkylamino, alkylthio, haloalkoxy, amino, aryl, heteroaryl, alkenyl, alkynyl, heterocyclyl, sulfhydryl, nitro, aryloxy, hydroxyalkoxy, alkanoyl, benzyl, cyclopropyl, phenyl, alkyl-C(=O)-, alkyl-C(=O)-NH-, formamide or alkoxyalkyl, etc.

The term "heterocyclyl" used in the present disclosure refers to a saturated or partially unsaturated ring system group containing one or more heteroatoms (O, S, or N), wherein the nitrogen and sulfur atoms are optionally oxidized and the nitrogen atoms are optionally quaternized as ring atoms. Unless otherwise specified, the ring system of " heterocycloalkyl" can be a monocyclic, bicyclic, spirocyclic, or polycyclic ring system. "Heterocycloalkyl" can be attached to the rest of a molecule through more than one cyclic carbon or heteroatom. Examples of "heterocycloalkyl" include, but are not limited to, pyrrolidine, piperidine, N-methylpiperidine, tetrahydroimidazole, pyrazolidine, butyrolactam, valerolactam, imidazolinone, hydantoin, dioxolane, phthalimide, piperidine, pyrimidine-2,4(1H,3H)-dione, 1,4-dioxane, morpholine, thiomorpholine, thiomorpholine-S-oxide, thiomorpholine-S,S-oxide, piperazine, pyran, pyridone, 3-pyrroline, thiopyran, pyrone, tetrahydrofuran, tetrahydrothiophene, quinine ring, 2-azaspiro[3.3]heptane, etc.

The term "alkylamino" used in the present disclosure refers to "N-alkylamino", in which the amino groups are independently substituted by one or two alkyl groups, wherein the alkyl group has the meaning as described in the present disclosure. In some embodiments, the alkylamino group is a lower-level alkylamino group with a C₁₋₆ alkyl group attached to a nitrogen atom. In other embodiments, the alkylamino group is a lower-level alkyl amino group with a C₁₋₃. Such examples include, but are not limited to, N-methylamino, N-ethylamino, and the like.

### Synthesis of compounds:

The preparation methods of the compound of formula (I) of the present disclosure are described in detail below, but these specific methods do not constitute any limitations to the present disclosure.

The compound of formula (I) described above can be synthesized using standard synthesis techniques or well-known techniques combined with the methods described herein. In addition, the solvents, temperatures, and other reaction conditions mentioned herein can be varied. The starting materials for the synthesis of compounds can be synthesized or obtained from commercial sources, such as, but not limited to, Aldrich Chemical Co. (Milwaukee, Wis.) or Sigma Chemical Co. (St. Louis, Mo.). The general method of compound preparation can be modified by using appropriate reagents and conditions to introduce different groups into the provided molecular formula.

In one aspect, the compounds described herein are according to methods well-known in the art. However, the conditions of the method, such as reactants, solvents, bases, amount of compounds used, reaction temperature, time required for the reaction, and so on, are not limited to the following explanations. The compounds of the present disclosure can also be conveniently prepared by optionally combining various synthesis methods described in this specification or known in the art, and such combinations can be easily performed by those skilled in the art to which the present disclosure belongs. In one aspect, the present disclosure also provides a method for preparing the compound represented by the general formula (I), which is prepared by using the following general reaction:

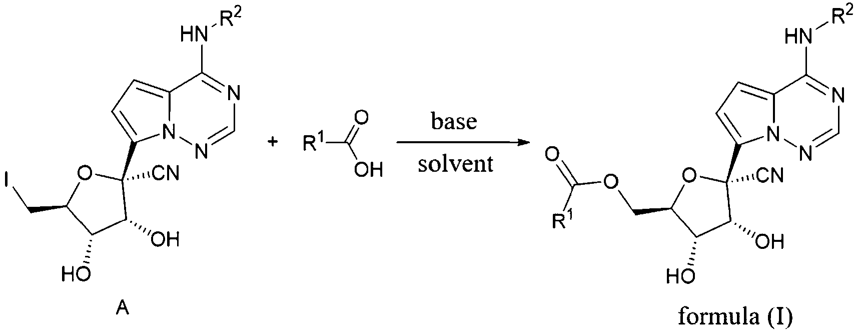

Wherein, the definitions of R¹ and R² are defined as mentioned above.

Compound A is used as the starting material and the compound of formula (I) is obtained through the nucleophilic substitution reaction.

The features mentioned above in the present disclosure or the features mentioned in the embodiments can be combined in any combination. All features disclosed in the specification of this case can be combined with any combination form, and each feature disclosed in the specification can be replaced by any alternative feature that serves the same, equivalent, or similar purpose. Therefore, unless otherwise specified, the disclosed features are only general examples of equivalent or similar features.

In the following description, various specific aspects, characteristics, and advantages of the above-mentioned compounds, methods, and pharmaceutical compositions will be elaborated in detail, so that the content of the present disclosure will become clear. It should be understood that the following detailed description and examples describe specific embodiments and are for reference only. After reading the description of the present disclosure, those skilled in the art may make various changes or modifications to the present disclosure, and these equivalent situations also fall within the scope defined by the present disclosure.

In all embodiments, ¹H-NMR was recorded using a Vian Mercury 400 nuclear magnetic resonance instrument, and the chemical shifts were expressed in δ (ppm); the silica gel used for separation without specified was 200-300 mesh, and the eluent ratios were all volume ratios.

The following abbreviations are used in the present disclosure: room temperature (RT, rt); aqueous solution (aq.); petroleum ether (PE); ethyl acetate (EA); dichloromethane (DCM); methanol (MeOH); methyl tert-butyl ether (MTBE); ethanol (EtOH); trifluoroacetic acid (TFA); equivalent (eq); gram/milligram (g/mg); moles/millimoles (mol/mmol); liter/milliliter (L/mL); minute (min (s)); hours (h, hr, hs); nitrogen (N₂); nuclear magnetic resonance (NMR); liquid chromatography-mass spectrometry (LC-MS); thin layer chromatography (TLC); preparative liquid chromatography (pre-HPLC).

### Example 1: Preparation of Compound 32

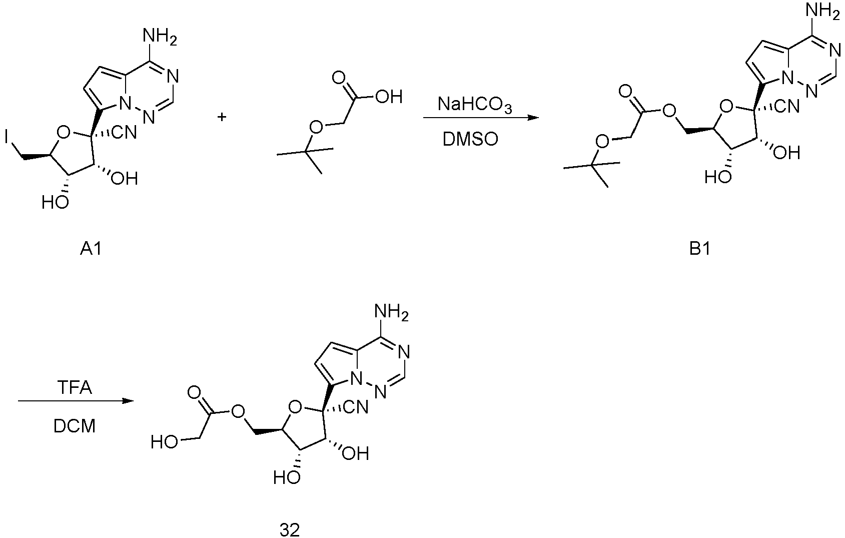

Step 1: 1.32g of tert-butoxyacetic acid and 0.84g of sodium bicarbonate were added to 30mL of DMSO, and the mixture was stirred at room temperature for 20 minutes until dissolved. Add 2.0g of raw material A1 at room temperature, the mixture was heated to 65°C, and kept at 65°C overnight. The raw material disappears and the product is generated. The reaction mixture was extracted with EA/water, the organic phase was washed with saturated brine, dried, concentrated, columned, and eluted with DCM/MeOH=30/1 to obtain 1.1g of white solid B1.

¹H NMR (400 MHz, DMSO-*d*₆) δ 8.05 - 7.86 (m, 3H), 6.92 (d, *J =* 4.4 Hz, 1H), 6.82 (d, *J =* 4.4 Hz, 1H), 6.36 (d, *J =* 6.0 Hz, 1H), 5.42 (d, *J =* 5.8 Hz, 1H), 4.68 (t, *J =* 5.4 Hz, 1H), 4.38 (d, *J =* 9.8 Hz, 1H), 4.28 - 4.14 (m, 2H), 4.00 (s, 2H), 3.96 - 3.89 (m, 1H), 1.12 (s, 9H).

Step 2: 500mg of raw material compound B1 was dissolved in 10mL of DCM, and 3mL of TFA was added dropwise under an ice bath, and the mixture was stirred at room temperature for 1 hour. LCMS showed that the reaction was complete and the product was produced. The reaction mixture was oncentrated at low temperature to remove the solvent, and then evaporated twice with DCM to obtain a colorless oily substance, and the oily substance was slurried with 10mL of MTBE to obtain a white solid, and then the white solid was slurried with 5mL of DCM twice. After concentrating and drying, white solid was obtained (600mg, trifluoroacetate of compound 32).

By the similar synthesis method, the compounds listed in Table 1 can be obtained:

**Table 1. Compound 1-106:**

| No. | Structure | MS (M+H)⁺ | No. | Structure | MS (M+H)⁺ |
|---|---|---|---|---|---|
| 1 | 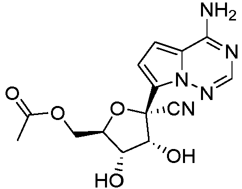 | 334.1 | 2 | 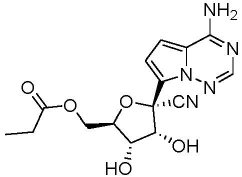 | 348.1 |
| 3 | 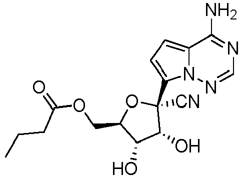 | 362.1 | 4 | 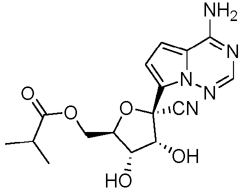 | 362.1 |
| 5 | 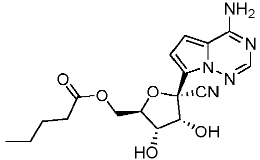 | 376.1 | 6 | 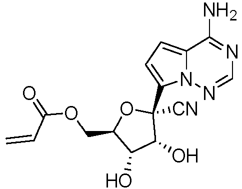 | 346.1 |
| 7 | 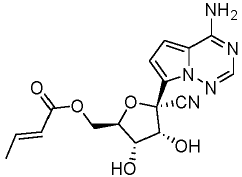 | 360.1 | 8 | 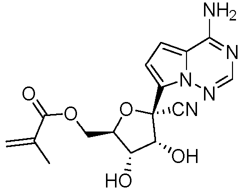 | 360.1 |
| 9 | 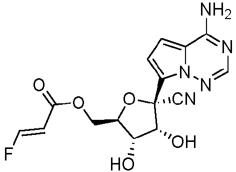 | 364.1 | 10 | 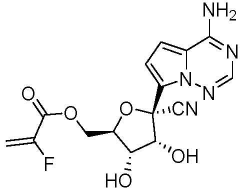 | 364.1 |
| 11 | 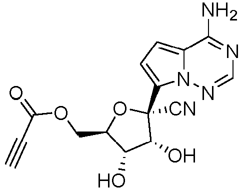 | 344.1 | 12 | 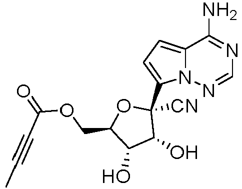 | 358.1 |
| 13 | 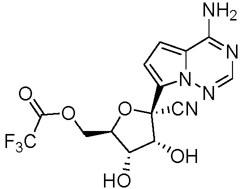 | 388.1 | 14 | 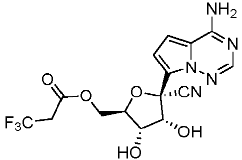 | 402.1 |
| 15 | 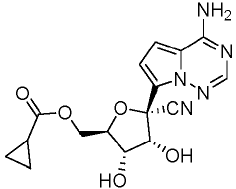 | 360.1 | 16 | 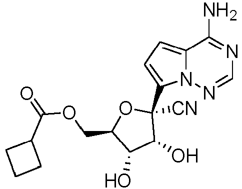 | 374.1 |
| 17 | 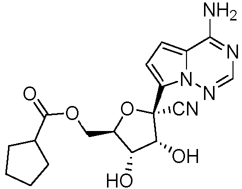 | 388.1 | 18 | 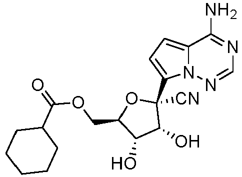 | 402.2 |
| 19 | 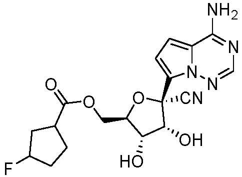 | 406.1 | 20 | 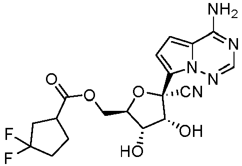 | 424.1 |
| 21 | 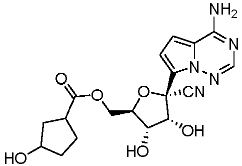 | 404.2 | 22 | 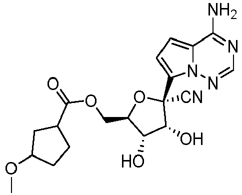 | 418.2 |
| 23 | 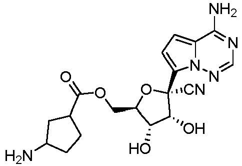 | 403.2 | 24 | 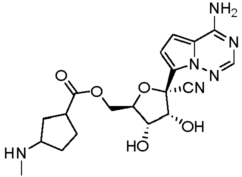 | 417.2 |
| 25 | 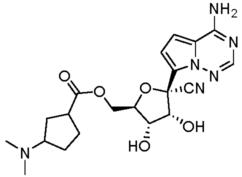 | 431.2 | 26 | 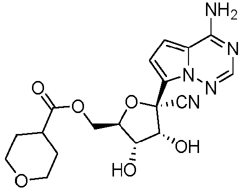 | 404.1 |
| 27 | 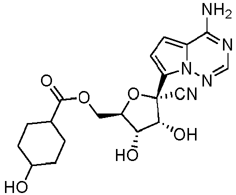 | 418.2 | 28 | 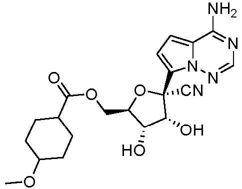 | 432.2 |
| 29 | 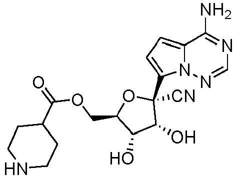 | 403.2 | 30 | 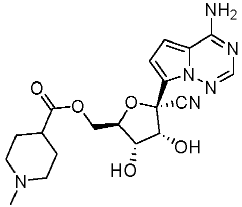 | 417.2 |
| 31 | 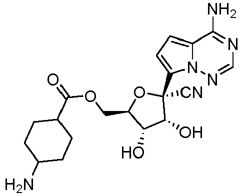 | 417.2 | 32 | 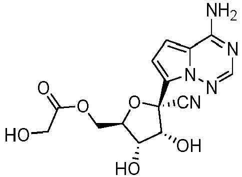 | 350.1 |
| 33 | 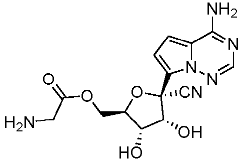 | 349.1 | 34 | 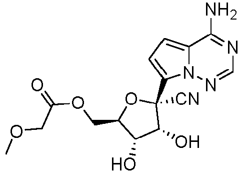 | 364.1 |
| 35 | 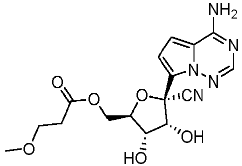 | 378.1 | 36 | 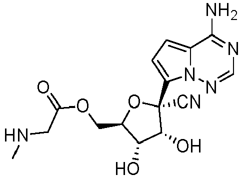 | 363.1 |
| 37 | 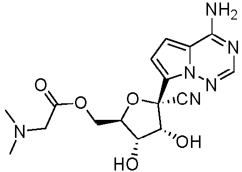 | 377.1 | 38 | 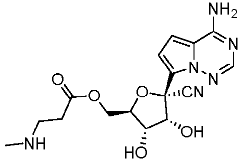 | 377.1 |
| 39 | 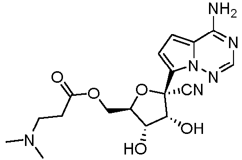 | 391.2 | 40 | 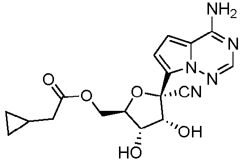 | 374.1 |
| 41 | 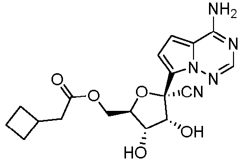 | 388.2 | 42 | 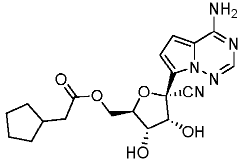 | 402.2 |
| 43 | 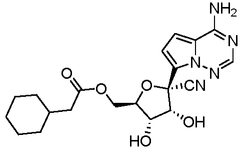 | 416.2 | 44 | 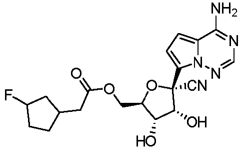 | 420.2 |
| 45 | 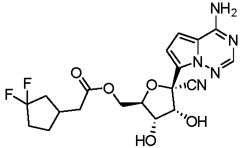 | 438.2 | 46 | 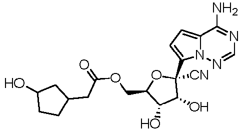 | 418.2 |
| 47 | 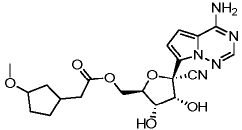 | 432.2 | 48 | 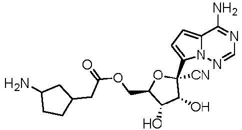 | 417.2 |
| 49 | 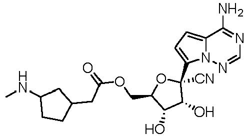 | 431.2 | 50 | 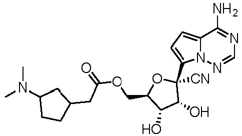 | 445.2 |
| 51 | 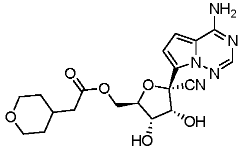 | 418.2 | 52 | 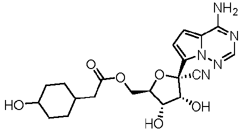 | 432.2 |
| 53 | 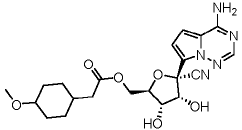 | 446.2 | 54 | 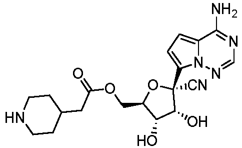 | 417.2 |
| 55 | 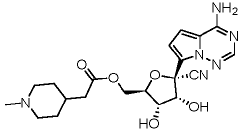 | 431.2 | 56 | 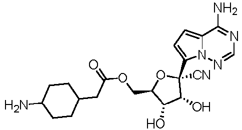 | 431.2 |
| 57 | 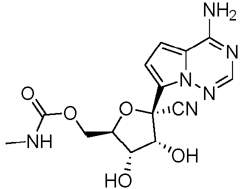 | 349.1 | 58 | 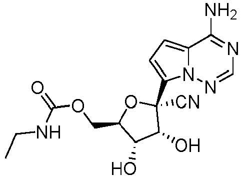 | 363.1 |
| 59 | 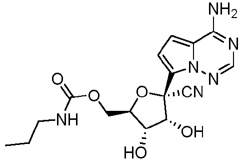 | 377.2 | 60 | 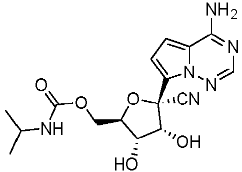 | 377.2 |
| 61 | 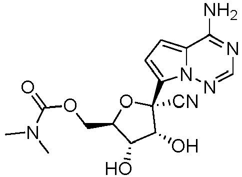 | 363.1 | 62 | 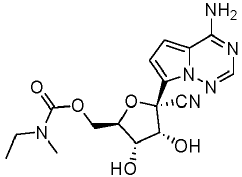 | 377.2 |
| 63 | 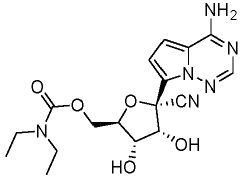 | 391.2 | 64 | 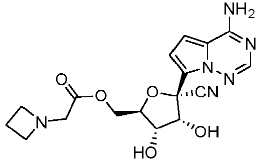 | 389.2 |
| 65 | 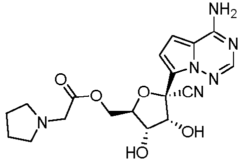 | 403.2 | 66 | 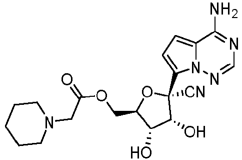 | 417.2 |
| 67 | 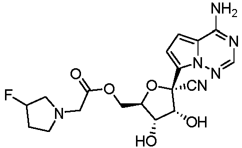 | 421.2 | 68 | 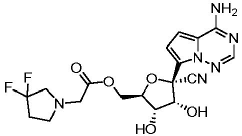 | 439.2 |
| 69 | 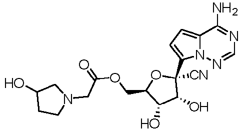 | 419.2 | 70 | 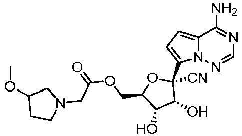 | 433.2 |
| 71 | 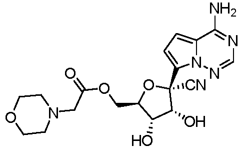 | 419.2 | 72 | 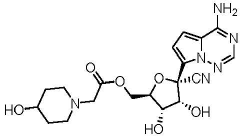 | 433.2 |
| 73 | 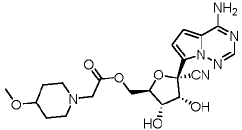 | 447.2 | 74 | 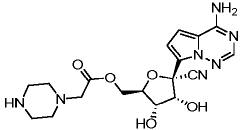 | 418.2 |
| 75 | 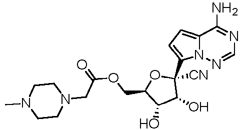 | 432.2 | 76 | 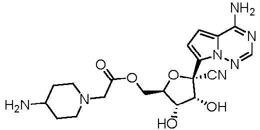 | 432.2 |
| 77 | 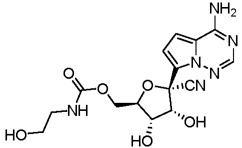 | 379.1 | 78 | 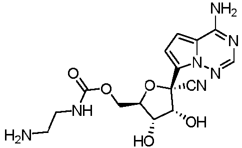 | 378.1 |
| 79 | 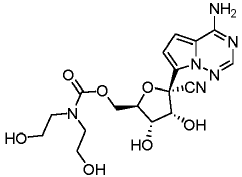 | 423.2 | 80 | 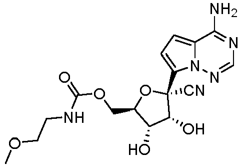 | 393.1 |
| 81 | 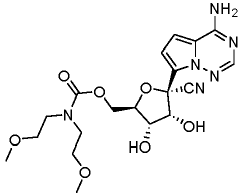 | 451.2 | 82 | 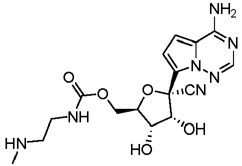 | 392.2 |
| 83 | 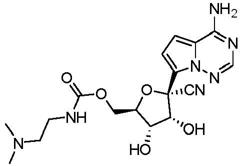 | 406.2 | 84 | 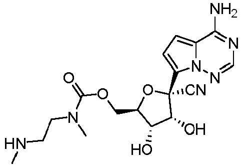 | 406.2 |
| 85 | 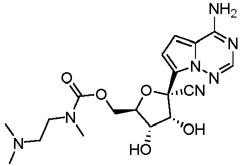 | 420.2 | 86 | 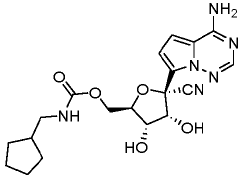 | 417.2 |
| 87 | 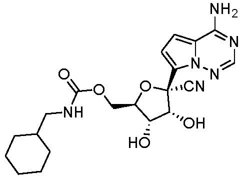 | 431.2 | 88 | 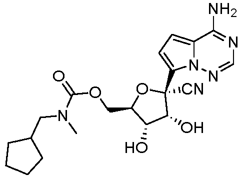 | 431.2 |
| 89 | 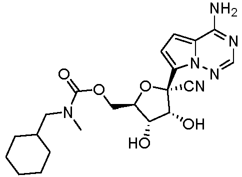 | 445.2 | 90 | 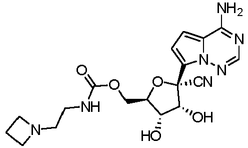 | 418.2 |
| 91 | 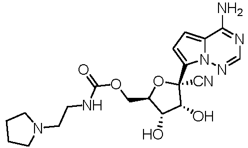 | 432.2 | 92 | 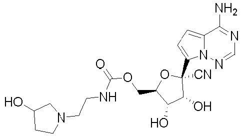 | 448.2 |
| 93 | 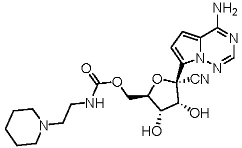 | 446.2 | 94 | 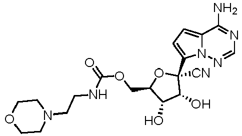 | 448.2 |
| 95 | 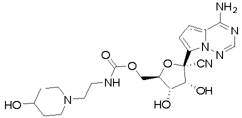 | 462.2 | 96 | 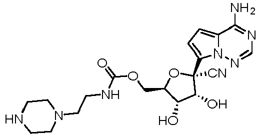 | 447.2 |
| 97 | 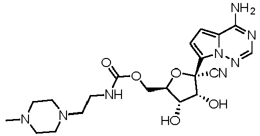 | 461.2 | 98 | 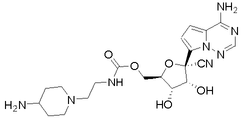 | 461.2 |

### ¹H-NMR data of representative compounds:

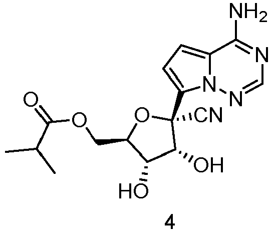

¹H NMR (500 MHz, DMSO-*d₆*) δ 7.93 (s, 3H), 6.91 (d, *J =* 4.5 Hz, 1H), 6.81 (d, *J* = 4.5 Hz, 1H), 6.33 (d, *J* = 6.0 Hz, 1H), 5.39 (d, *J* = 5.8 Hz, 1H), 4.71 - 4.68 (m, 1H), 4.31 (dd, *J =* 12.0, 2.9 Hz, 1H), 4.26 - 4.21 (m, 1H), 4.17 (dd, *J =* 12.1, 5.2 Hz, 1H), 3.98 - 3.93 (m, 1H), 2.56 - 2.52 (m, 1H), 1.08 - 1.03 (m, 6H).

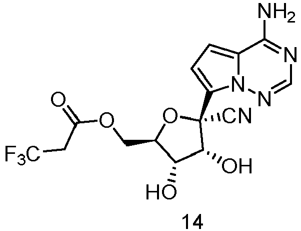

¹H NMR (500 MHz, DMSO-*d₆*) δ 8.02 - 7.78 (m, 3H), 6.91 (d, *J =* 4.6 Hz, 1H), 6.79 (d, *J =* 4.5 Hz, 1H), 6.34 (d, *J =* 6.0 Hz, 1H), 5.38 (d, *J =* 6.0 Hz, 1H), 4.67 (t, *J=* 5.4 Hz, 1H), 4.38 - 4.29 (m, 1H), 4.26 - 4.12 (m, 2H), 3.98 - 3.88 (m, 1H), 3.21 - 3.09 (m, 1H), 2.20 - 2.06 (m, 4H), 1.98 - 1.86 (m, 1H), 1.84 - 1.73 (m, 1H).

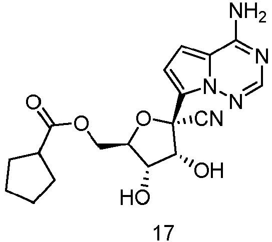

¹H NMR (500 MHz, DMSO-*d₆*) δ 8.04 - 7.78 (m, 3H), 6.92 (d, *J =* 4.5 Hz, 1H), 6.81 (d, *J =* 4.6 Hz, 1H), 6.34 (d, *J =* 6.0 Hz, 1H), 5.39 (d, *J =* 5.9 Hz, 1H), 4.74 - 4.65 (m, 1H), 4.36 - 4.28 (m, 1H), 4.27 - 4.21 (m, 1H), 4.20 - 4.14 (m, 1H), 4.00 - 3.91 (m, 1H), 2.76 - 2.66 (m, 1H), 1.85 - 1.71 (m, 2H), 1.69 - 1.45 (m, 6H).

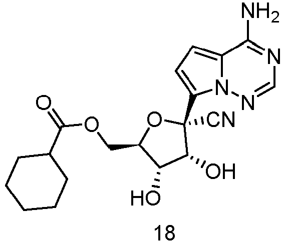

¹H NMR (500 MHz, DMSO-*d*₆) δ 8.06 - 7.79 (m, 3H), 6.93 (d, *J =* 4.5 Hz, 1H), 6.82 (d, *J =* 4.5 Hz, 1H), 6.34 (d, *J =* 6.0 Hz, 1H), 5.38 (d, *J =* 5.9 Hz, 1H), 4.74 - 4.68 (m, 1H), 4.31 (dd, *J =* 12.2, 2.9 Hz, 1H), 4.26 - 4.21 (m, 1H), 4.15 (dd, *J =* 12.2, 5.0 Hz, 1H), 4.00 - 3.94 (m, 1H), 2.30 - 2.22 (m, 1H), 1.81 - 1.54 (m, 5H), 1.34 - 1.11 (m, 5H).

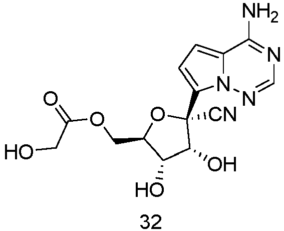

¹H NMR (400 MHz, DMSO-*d*₆) δ 8.05 - 7.77 (m, 3H), 6.91 (d, *J =* 3.4 Hz, 1H), 6.81 (d, *J =* 3.4 Hz, 1H), 6.29 (d, *J =* 5.9 Hz, 1H), 5.44 - 5.31 (m, 2H), 4.76 - 4.65 (m, 1H), 4.44 - 4.33 (m, 1H), 4.30 - 4.16 (m, 2H), 4.06 - 3.88 (m, 3H).

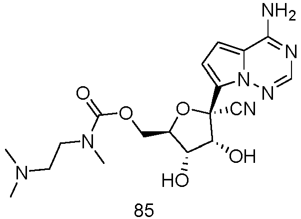

¹H NMR (500 MHz, DMSO-*d*₆) δ8.02 - 7.78 (s, 3H), 6.90 (d, *J =* 4.5 Hz, 1H), 6.78 (d, *J =* 4.5 Hz, 1H), 6.30 -6.24(m, 1H), 5.39 - 5.31 (m, 1H), 4.73 - 4.66 (m, 1H), 4.36 - 4.18 (m, 2H), 4.16 -4.07 (m, 1H), 4.00 - 3.94 (m, 1H), 3.28 - 3.17 (m, 2H), 2.85-2.74 (m, 3H), 2.35 - 2.24 (m, 2H), 2.14 (s, 3H), 2.06 (s, 3H).

### Example 2: Solubility Test

Experimental method: Refer to the high-performance liquid chromatography method (Chinese Pharmacopoeia, 2020 Edition, Part Four, General Rule 0512) for determination.
a. Take approximately 1-2mg of the compound, and place it in a 10ml volumetric flask. Add 1ml of water and vigorously shake for 30 seconds every 5 minutes. Observe for 30 minutes. The sample is not completely dissolved. Filter through a 0.45µm filter membrane, and take the remaining filtrate as the test solution.
b. Take approximately amount of the reference substance, add solvent (DMSO: methanol = 10:90) to dissolve and dilute it quantitatively to produce a solution containing 0.1mg per 1ml as the reference substance solution.
c. Accurately measure 20µl of each of the reference substance and test solution, and inject them into the liquid chromatograph respectively. Calculate the peak area according to the external standard method. The obtained data are shown in Table 2. Calculation formula: $solubility = \frac{{A}_{T} \times {W}_{R}}{{A}_{R} \times {V}_{R}} \times S$. In the formula: A_{T}: the main peak area of the test solution; A_{R}: the main peak area of reference substance solution; W_{R}: weighing amount of reference substance solution, mg; V_{R}: dilution volume of reference substance solution, ml; S: content of reference substance solution, %.
d. Chromatographic conditions: Using octadecylsilane bonded silica gel as the filling agent (Welch Ultimate XB-C18, 4.6 × 150mm, suitable for 5um columns). Use 0.05% H₃PO₄ solution (take 0.5ml of H₃PO₄ and dilute to 1000ml with water) as mobile phase A, use acetonitrile as mobile phase B, and perform linear gradient elution as shown in the following table. The column temperature is 30°C, and the detection wavelength is 254nm.

**Table 2. Solubility of Compounds of the Present Disclosure in Water**

| Time (min) | Phase A (%) | Phase B (%) |
|---|---|---|
| 0 | 95 | 5 |
| 8 | 80 | 20 |
| 13 | 50 | 50 |
| 23 | 5 | 95 |
| 26 | 5 | 95 |
| 26.1 | 95 | 5 |
| 31 | 95 | 5 |

| No. | Solubility | No. | Solubility | No. | Solubility | No. | Solubility |
|---|---|---|---|---|---|---|---|
| 1 | ++ | 2 | ++ | 4 | ++ | 6 | ++ |
| 11 | ++ | 13 | ++ | 15 | ++ | 26 | ++ |
| 30 | +++ | 32 | ++ | 33 | +++ | 35 | ++ |
| 39 | +++ | 40 | ++ | 55 | +++ | 61 | ++ |
| 79 | +++ | 81 | ++ | 85 | +++ | 88 | ++ |
| 97 | +++ | GS-441524 | 0.05mg/mL | | | | |

| | | | | | | | |
|---|---|---|---|---|---|---|---|
| Note: "+" represents 1 ≤ the solubility of the compound of the present disclosure/the solubility of GS-441524 <10; "++" represents 10 ≤ the solubility of the compound of the present disclosure/the solubility of GS-441524 <100; "+++" represents the solubility of the compound of the present disclosure/the solubility of GS-441524 ≥ 100. | | | | | | | |

Compared with GS-441524, the compounds of the present disclosure have higher solubility in water. Therefore, the compounds of the present disclosure are more readily absorbed by subjects.

### Example 3: Pharmacodynamic Experiment of Compounds 1, 32, and 85 on Feline Infectious Peritonitis

### Experimental method:

15mg of compounds 1, 32, and 85 was dissolved separately in 5 mL of solution (water: propylene glycol: PEG = 2:1:1) to prepare an injection solution for injection administration. The injection can be stored at room temperature. Use a 2 mL disposable veterinary syringe to draw the corresponding volume of the injection solution and expel air upwards. When injecting, choose a site with soft skin, loose subcutaneous tissue, and fewer blood vessels, such as the neck or medial thigh, for subcutaneous injection. The dosage of administration is 2 mg/kg body weight once a day. Blood samples are collected every three days and blood routine and corresponding biochemical indicators are measured. The end of the experiment is marked when the cat's biochemical indicators return to normal. The indicator data measured in the experiment includes body weight data, white blood cell data, and data of the ratio of Albumin to Globulin. The experimental subjects for each compound are 15 cats diagnosed with feline infectious peritonitis.

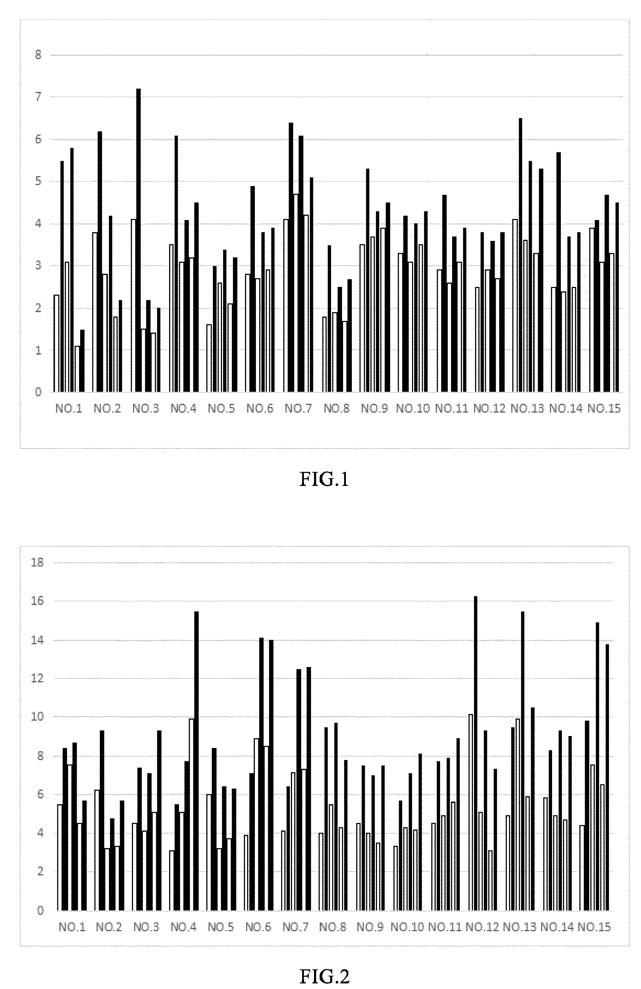

Figure 1 shows the changes in body weight of 3 groups of 15 cats of each group with feline infectious peritonitis before and after treatment with aqueous injection formulated with compounds 1, 32, and 85 respectively (vertical axis unit: kg, white columns represent before treatment, black columns represent after treatment, the first and second columns of each group are compound 1, the third and fourth columns are compound 32, and the fifth and sixth columns are compound 85).

From Figure 1, it can be seen that the weight changes of cats treated with aqueous injection of compounds 1, 32, and 85 two weeks before and after treatment. The weights of cats after treatment were significantly raised.

Figure 2 shows the changes in the number of white blood cells of 15 experimental cats two weeks before and after treatment with aqueous injection formulated with compounds 1, 32, and 85 respectively (vertical axis unit: 1×10⁹ cells/L, white columns represent before treatment, black columns represent after treatment, the first and second columns of each group are compound 1, the third and fourth columns are compound 32, and the fifth and sixth columns are for compound 85).

From Figure 2, it can be seen that the number of white blood cells of cats treated with aqueous injection of compounds 1, 32, and 85 was significantly increased.

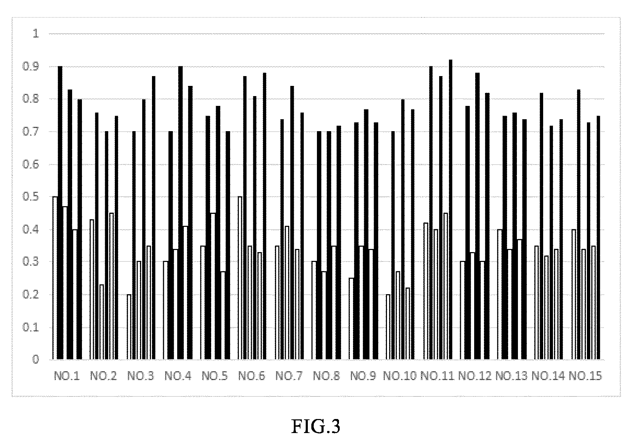

Figure 3 shows the changes in the ratio of Albumin to Globulin (A/G) of 15 experimental cats two weeks before and after treatment with aqueous injection formulated with compounds 1, 32, and 85 respectively (white columns represent before treatment, black columns represent after treatment, the first and second columns of each group are compound 1, the third and fourth columns are compound 32, and the fifth and sixth columns are compound 85).

From Figure 3, it can be seen that the ratio of Albumin to Globulin of 15 cats with feline infectious peritonitis corresponding to compounds 1, 32, and 85 reached normal values after two weeks of treatment with aqueous injection formulated with compounds 1, 32, and 85.

In this experiment, 3 groups of 15 cats of each group with feline infectious peritonitis were cured and without recurrence. The series of compounds reported in the present disclosure have excellent water solubility, which is significantly different from the traditional GS441524 oil-based injections. Its absorption efficiency is higher and is easier to disperse in the body. The dosage is significantly lower than traditional injections.

### Example 4: In Vitro Anti Feline Infectious Peritonitis Virus (FIPV) Activity of Compounds

### Experimental method:

Well-growing CRFK cells were inoculated on a 96-well plate. When the cells grow to 80%-90% confluence, 0.1 MOI Feline Infectious Peritonitis Virus (FIPV) and different concentrations of test compounds (0.3125 µM, 0.625 µM, 1.25 µM, 2.5 µM, 5 µM, 10 µM, 20 µM, and 40 µM) were added to each well. Simultaneously set up cell control and virus control with 1% DMSO each well. After being incubated at 37 °C for 72 hours, the cells were treated with CellTier-Glo luminescence assay cell viability detection reagents (Promega, Madison, WI, USA), and the half effective concentration (EC50) of the compounds against virus inhibition was calculated by GraphPad prism 7 software.

**Table 3. In Vitro Inhibitory Activity of Compounds of The Present Disclosure Against Feline Infectious Peritonitis Virus (FIPV)**

| Compound | 4 | 14 | 16 | 18 | 32 |
|---|---|---|---|---|---|
| EC₅₀ (µM) | 0.65 | 2.95 | 0.81 | 0.35 | 6.21 |

As shown in Table 3, compounds 4, 14, 16, 18, and 32 of the present disclosure can inhibit the replication of feline infectious peritonitis virus (FIPV) in vitro. Where compounds 4, 16, and 18 have significant antiviral activity, with EC50 values of 0.65 µM, 0.81 µM, and 0.35 µM respectively.

## Claims

1. A compound represented by formula (I) or a pharmaceutically acceptable salt thereof: 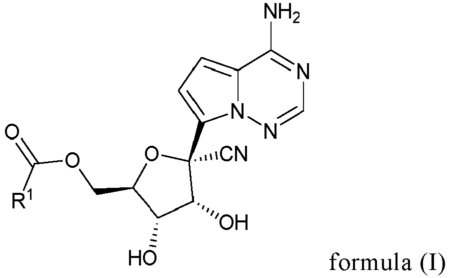 wherein,
the compound has one of the following structures:

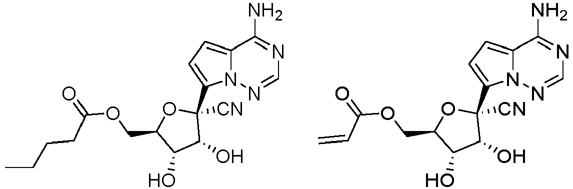

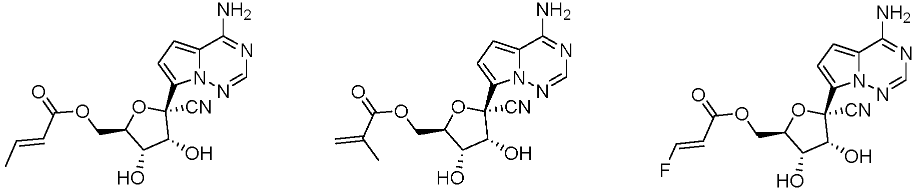

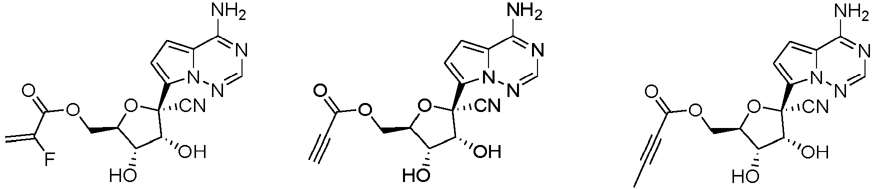

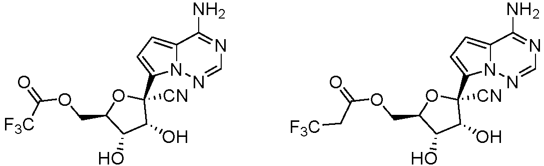

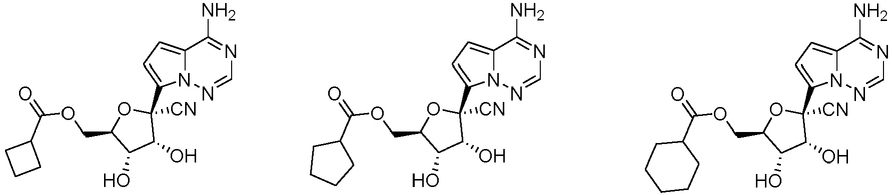

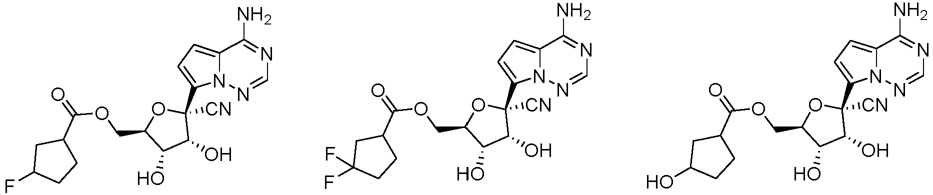

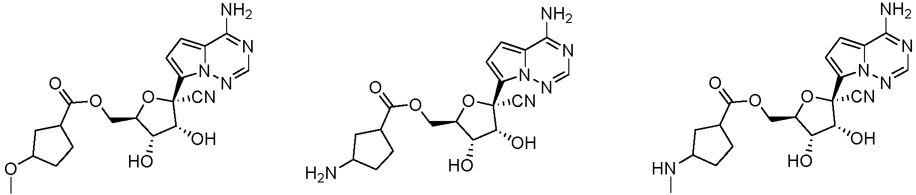

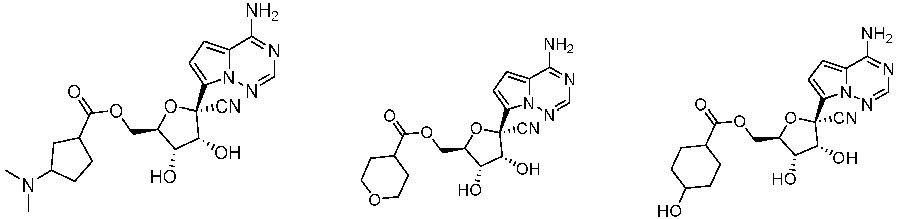

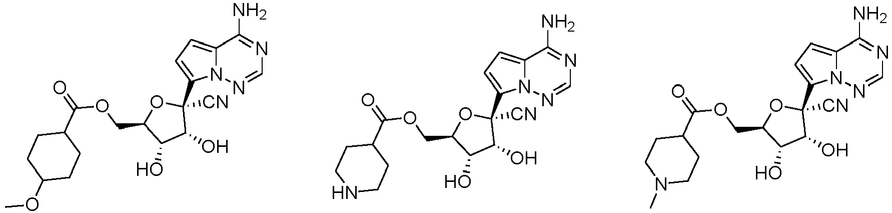

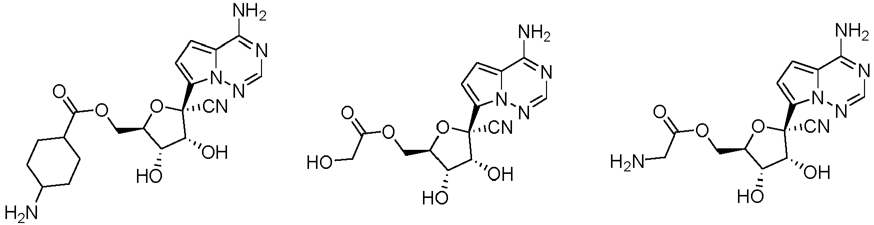

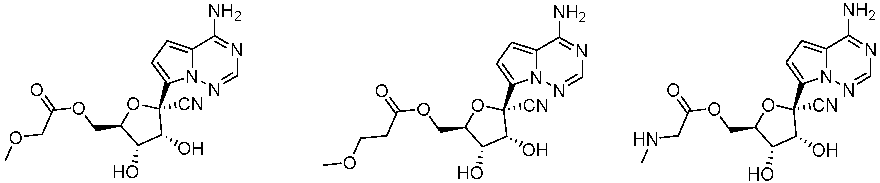

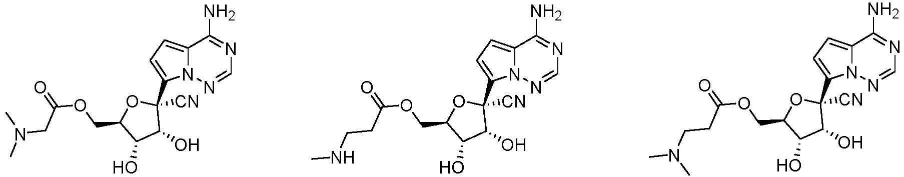

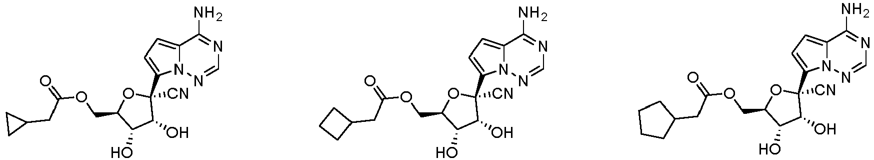

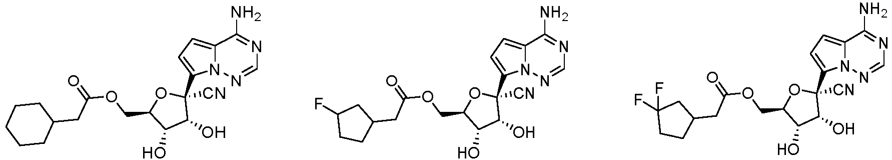

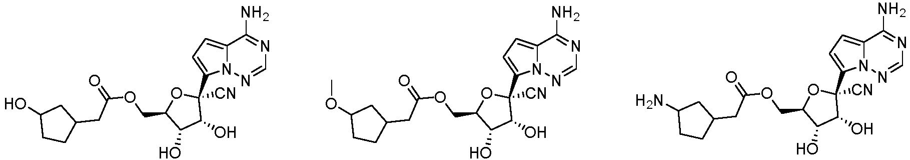

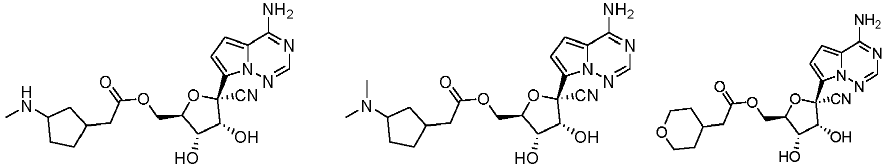

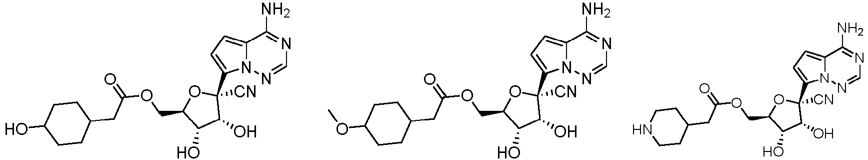

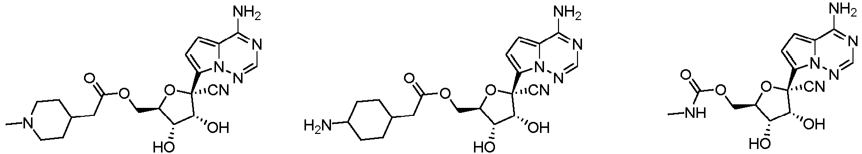

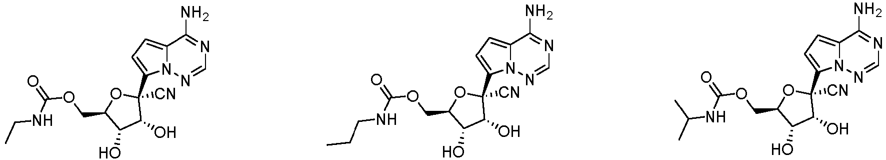

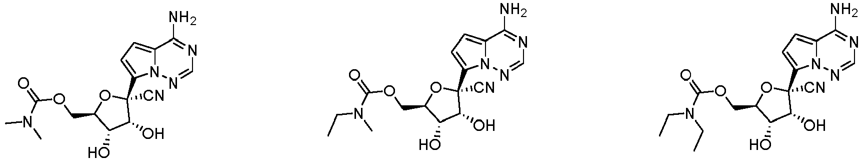

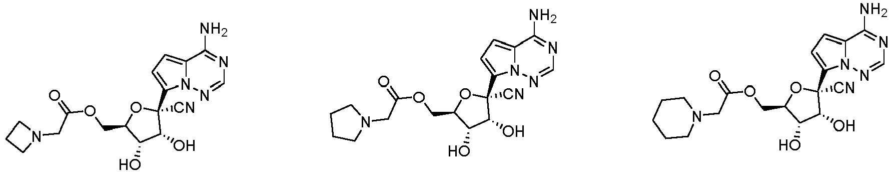

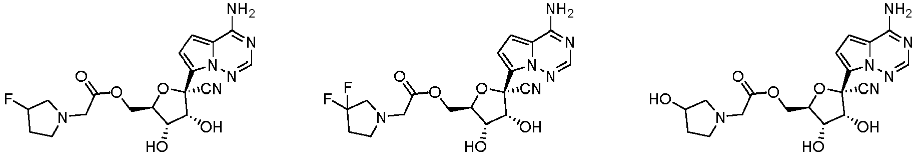

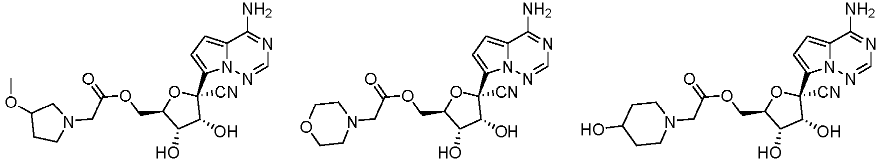

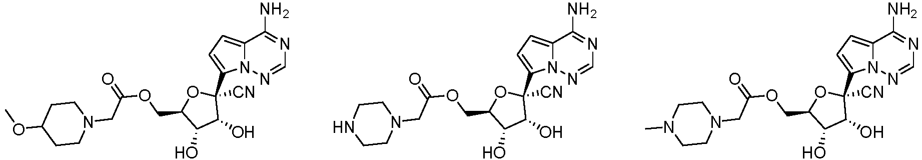

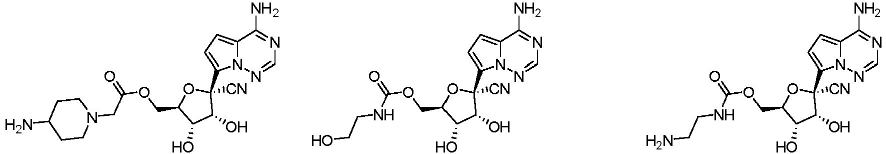

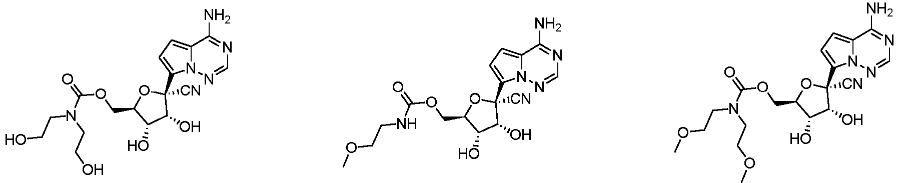

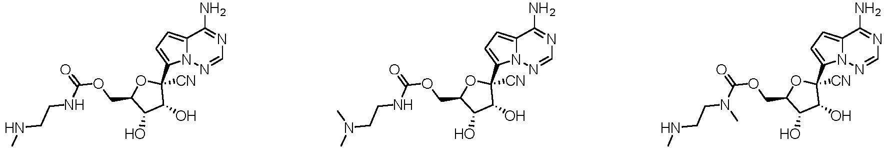

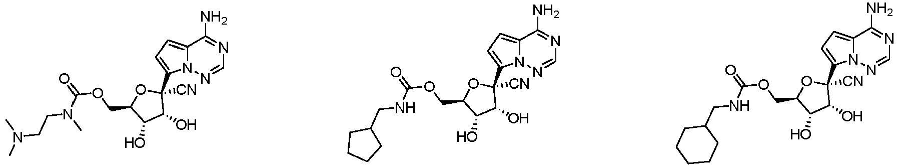

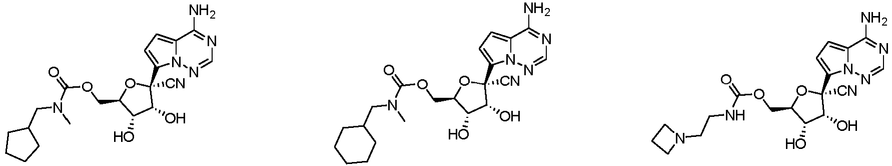

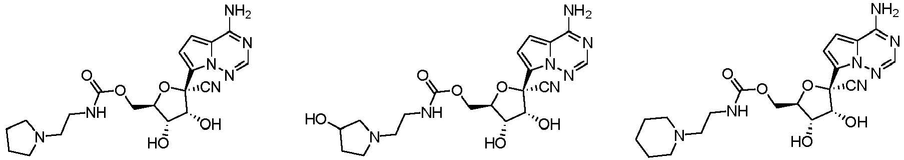

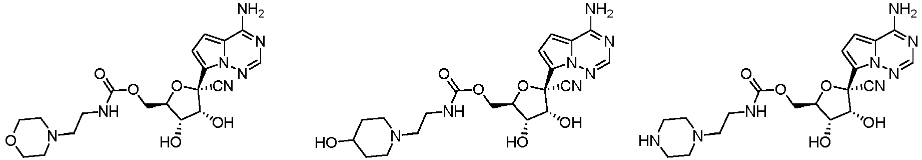

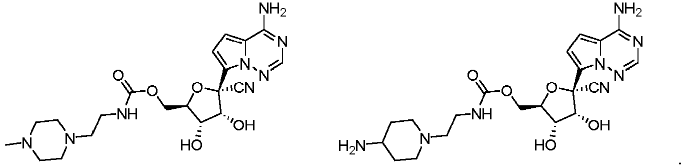

2. A pharmaceutical composition, wherein it contains pharmaceutically acceptable excipients or carriers, and the compound or the pharmaceutically acceptable salt thereof according to claim 1 as an active ingredient, wherein the active ingredient comprises each optical isomer, each crystal form, hydrate or solvate thereof.

3. The compound or the pharmaceutically acceptable salt thereof according to claim 1 for use in the treatment of diseases caused by feline coronavirus infection.

4. The pharmaceutical composition according to claim 2 for use in the treatment of diseases caused by feline coronavirus infection.

5. The compound or the pharmaceutically acceptable salt thereof for use according to claim 3, wherein the disease caused by the feline coronavirus infection is feline infectious peritonitis.

6. The pharmaceutical composition for use according to claim 4, wherein the disease caused by the feline coronavirus infection is feline infectious peritonitis.

## Patentansprüche

1. Verbindung, dargestellt durch die Formel (I), oder ein pharmazeutisch verträgliches Salz davon: 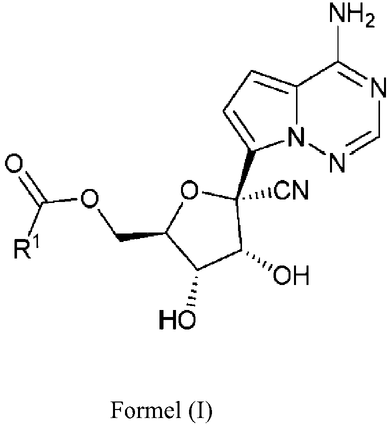 wobei die Verbindung eine der folgenden Strukturen aufweist:

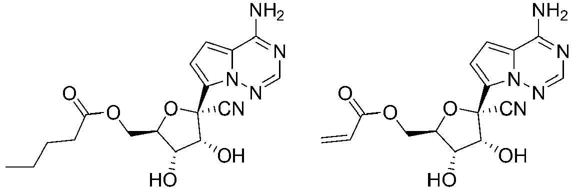

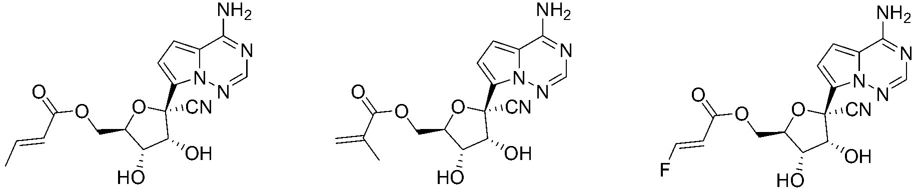

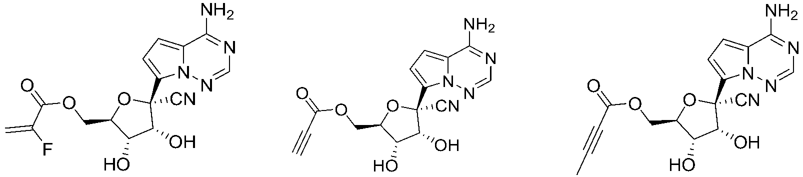

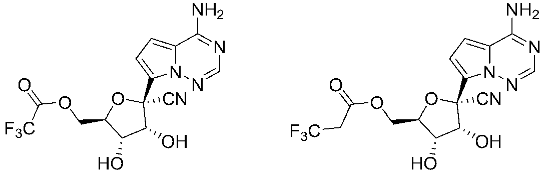

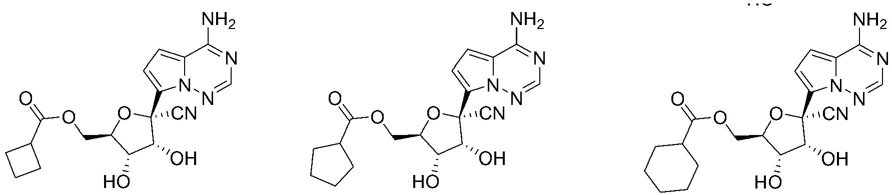

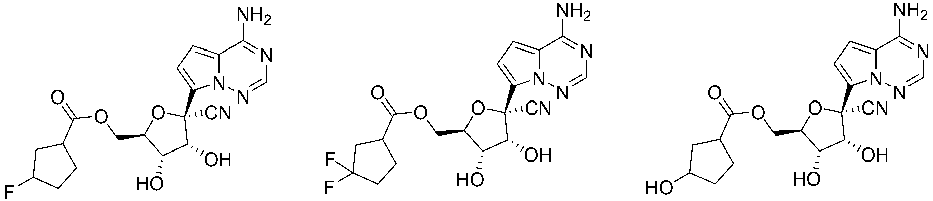

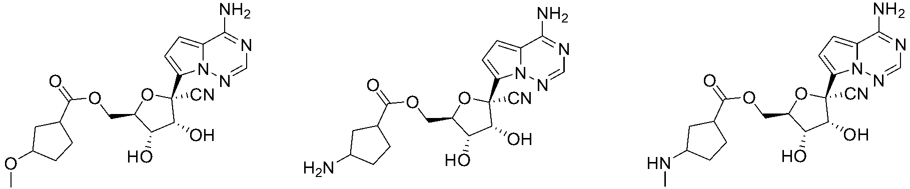

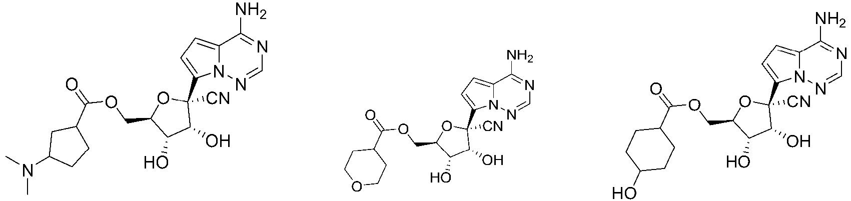

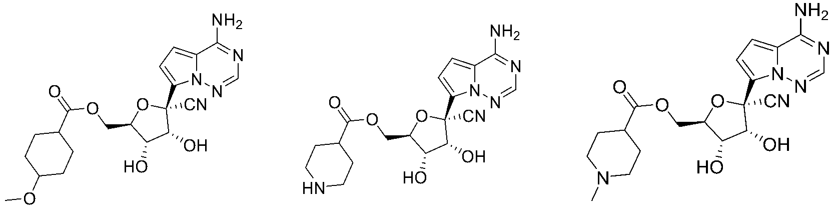

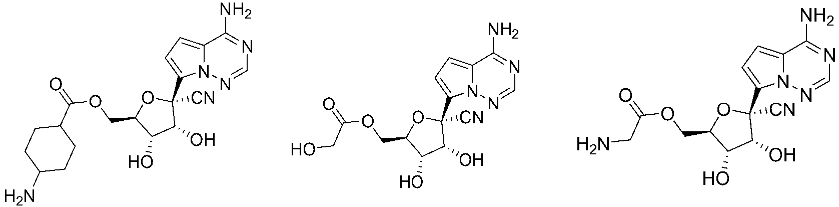

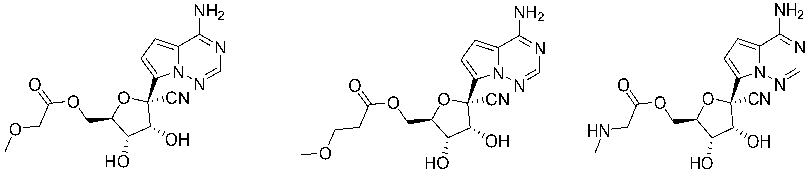

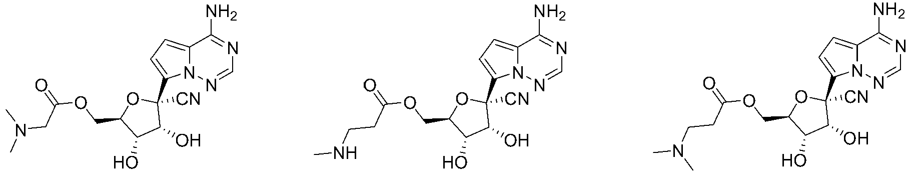

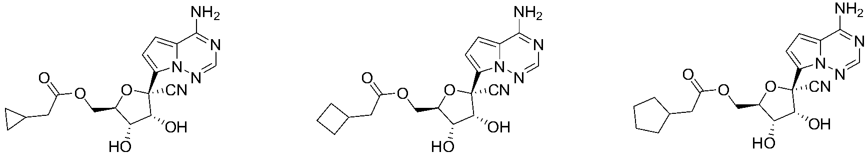

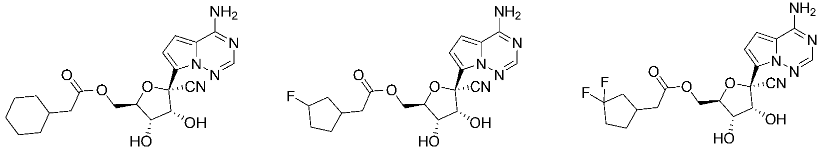

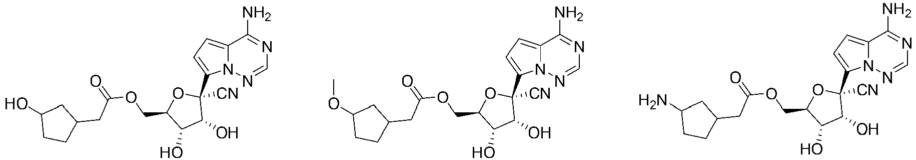

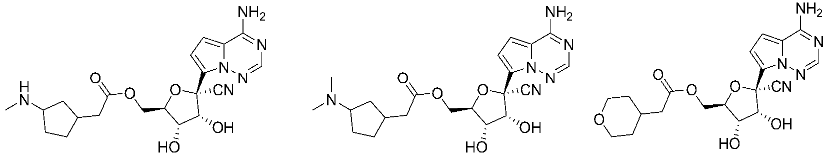

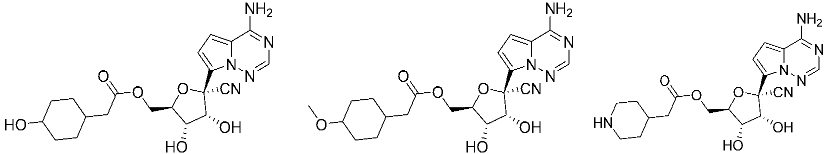

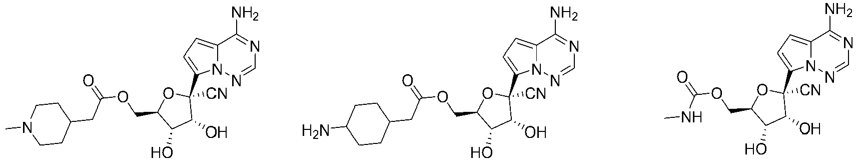

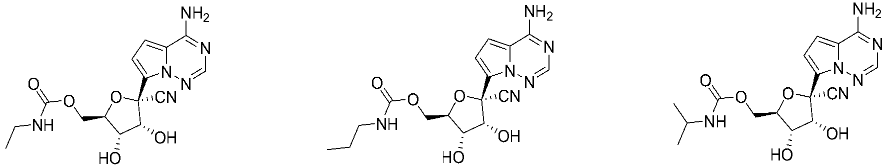

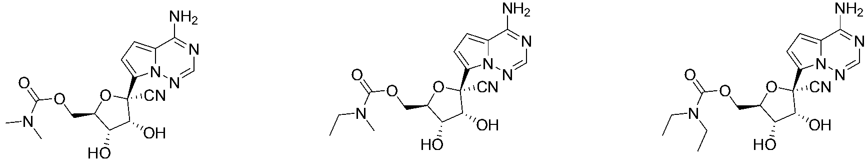

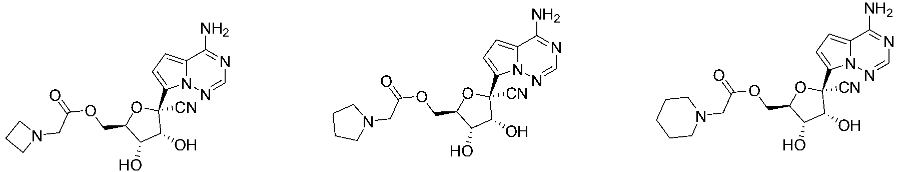

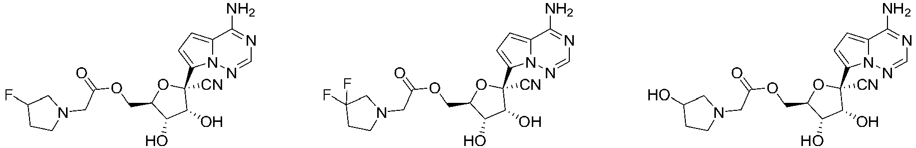

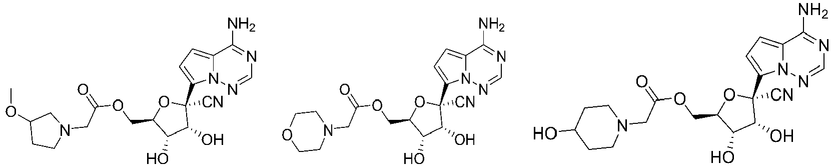

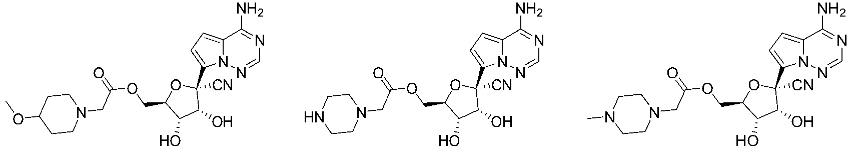

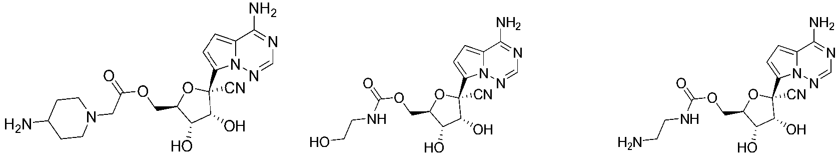

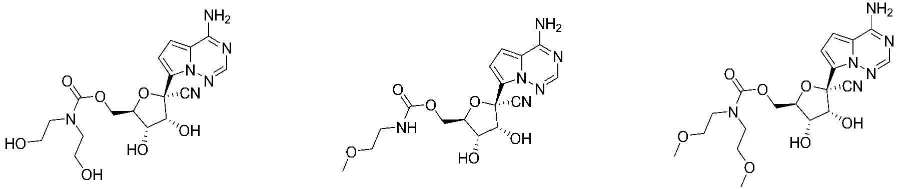

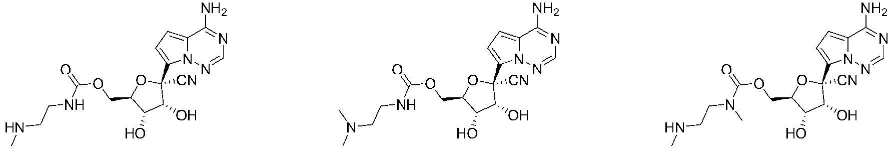

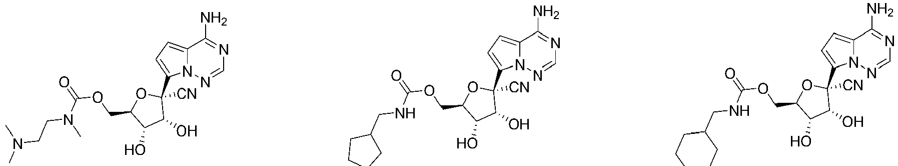

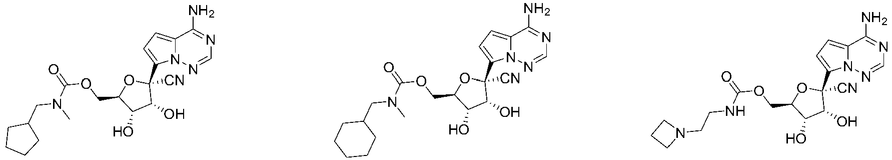

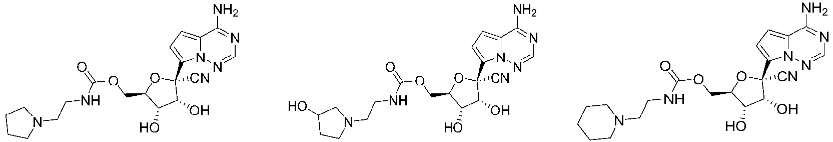

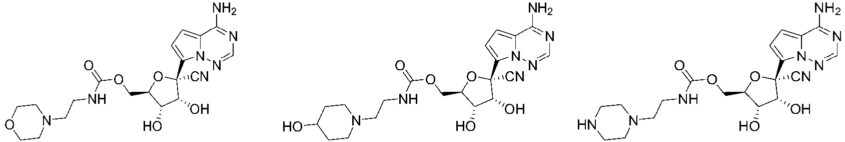

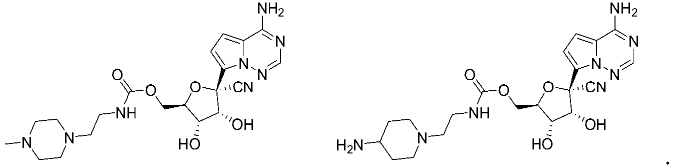

2. Pharmazeutische Zusammensetzung, enthaltend pharmazeutisch verträgliche Hilfsstoffe oder Träger und die Verbindung oder das pharmazeutisch verträgliche Salz davon nach Anspruch 1 als Wirkstoff, wobei der Wirkstoff jedes optische Isomer, jede Kristallform, Hydrat oder Solvat davon umfasst.

3. Verbindung oder pharmazeutisch verträgliches Salz davon nach Anspruch 1 zur Verwendung bei der Behandlung von Krankheiten, die durch eine Infektion mit felinem Coronavirus verursacht werden.

4. Pharmazeutische Zusammensetzung nach Anspruch 2 zur Verwendung bei der Behandlung von Krankheiten, die durch eine Infektion mit felinem Coronavirus verursacht werden.

5. Verbindung oder pharmazeutisch verträgliches Salz davon zur Verwendung nach Anspruch 3, wobei die durch die Infektion mit felinem Coronavirus verursachte Krankheit die feline infektiöse Peritonitis (FIP) ist.

6. Pharmazeutische Zusammensetzung zur Verwendung nach Anspruch 4, wobei die durch die Infektion mit felinem Coronavirus verursachte Krankheit die feline infektiöse Peritonitis ist.

## Revendications

1. Composé représenté par la formule (I) ou un sel pharmaceutiquement acceptable de celui-ci : 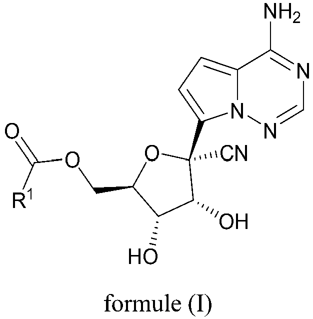 dans lequel,
le composé présente l'une des structures suivantes :

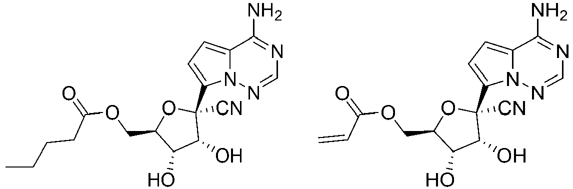

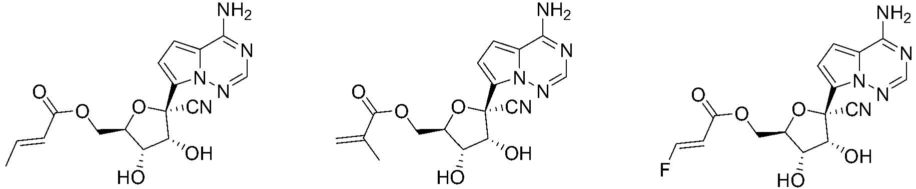

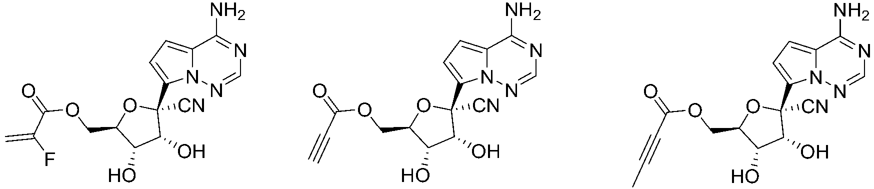

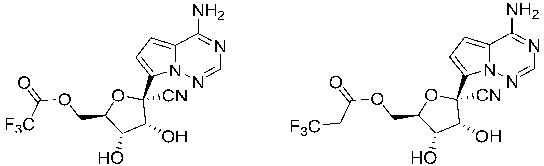

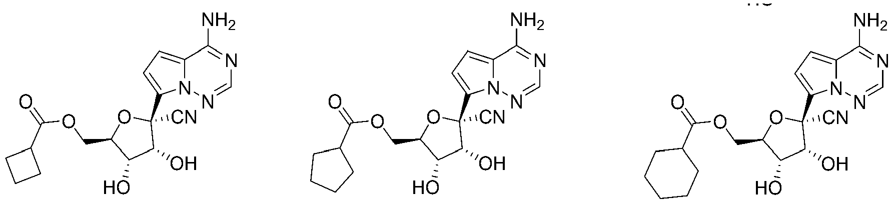

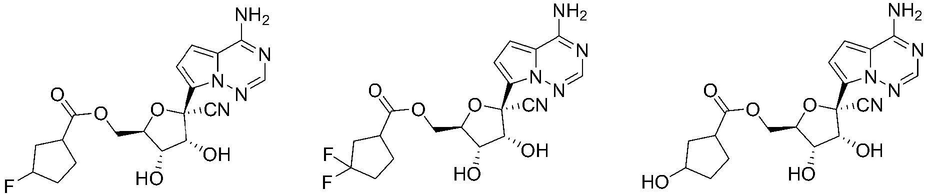

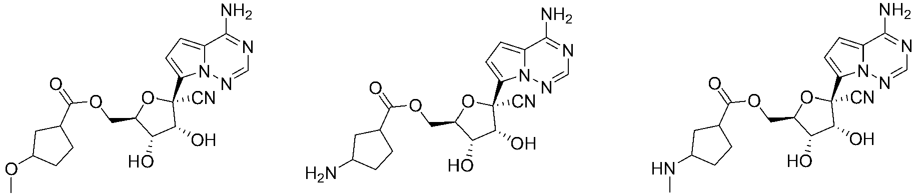

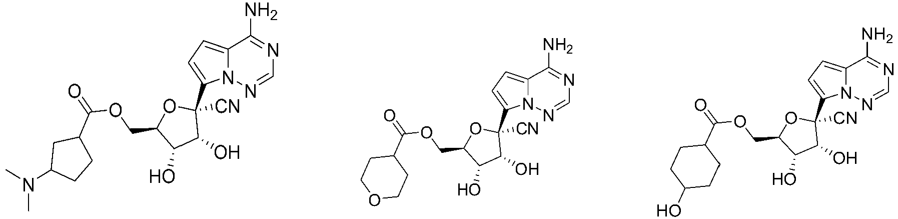

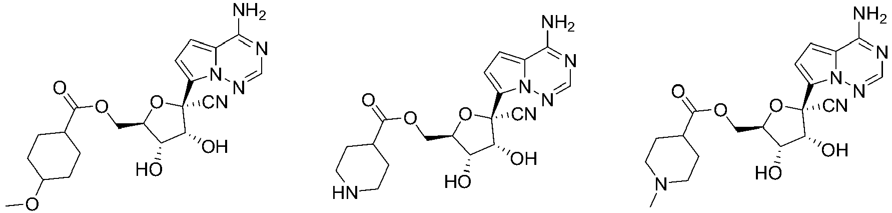

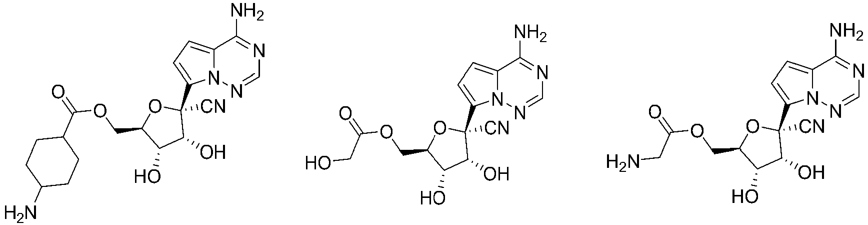

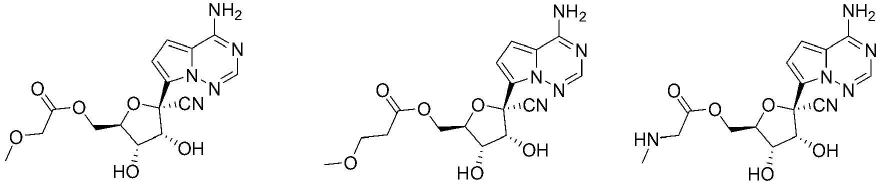

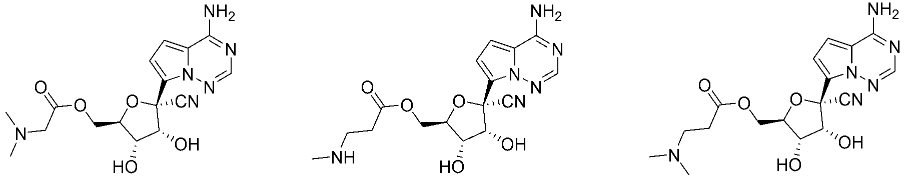

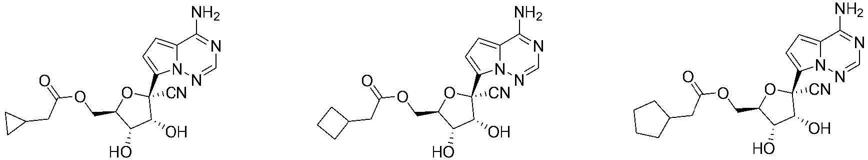

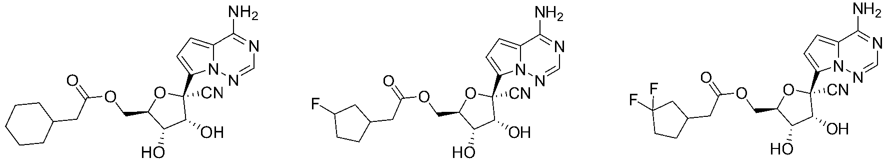

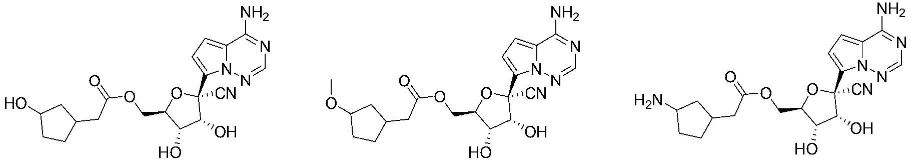

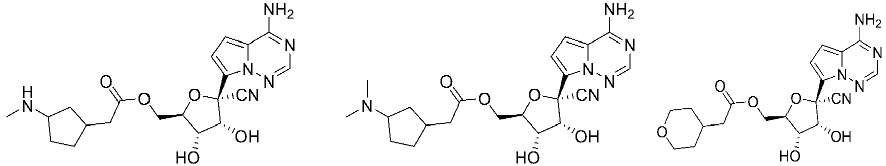

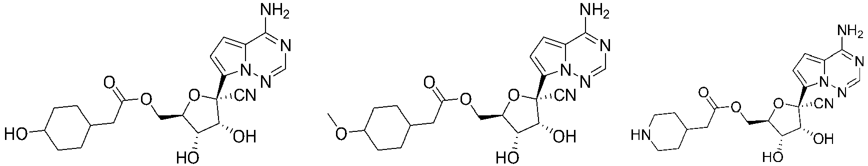

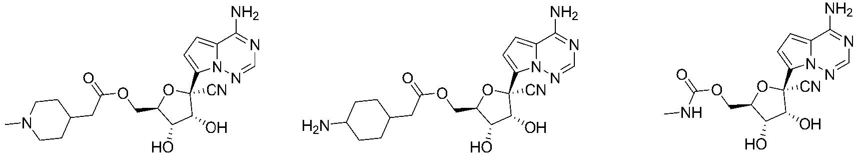

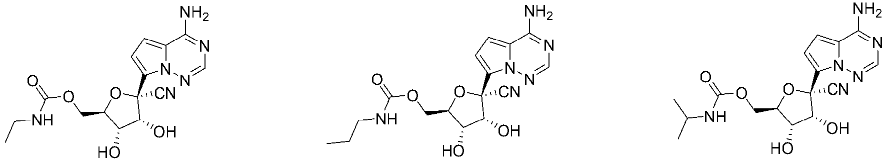

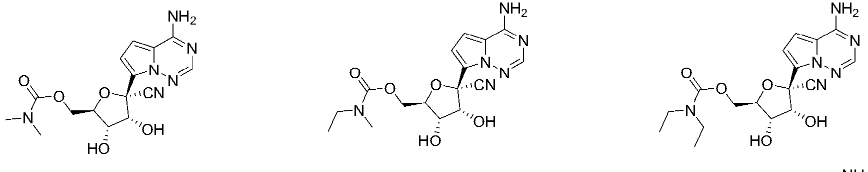

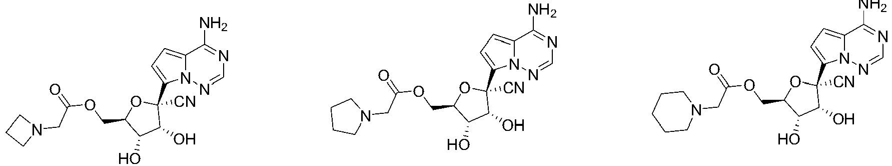

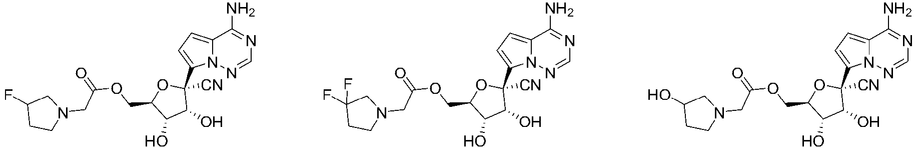

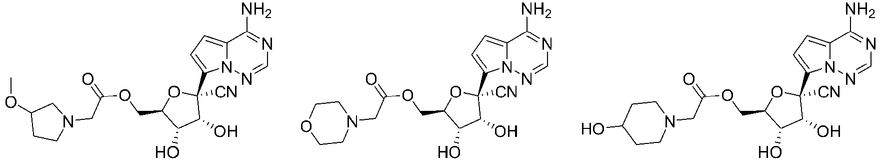

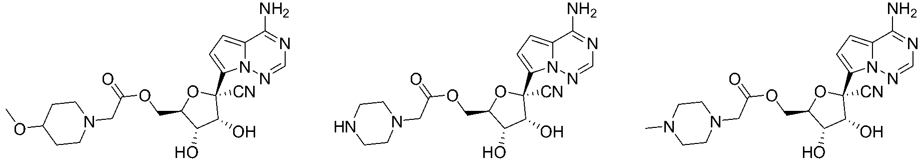

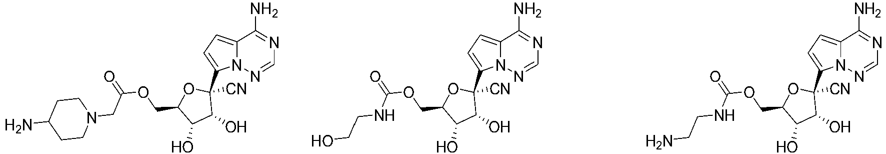

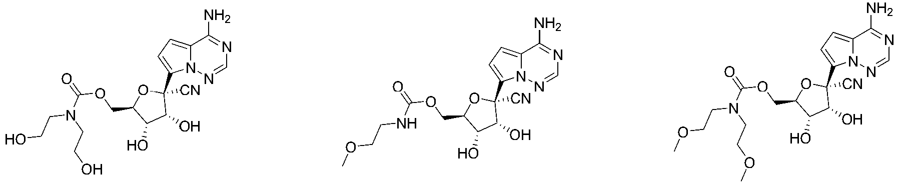

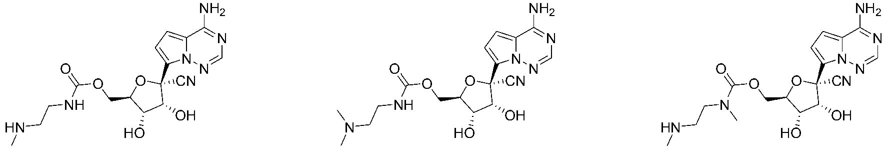

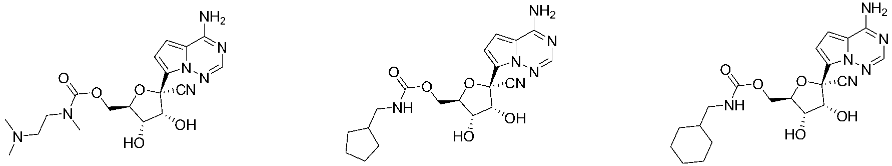

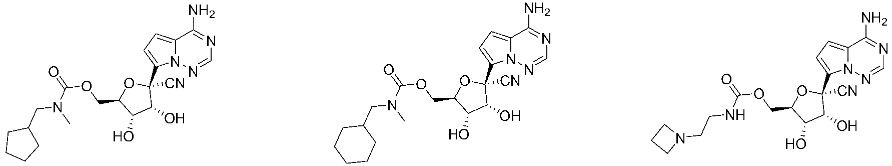

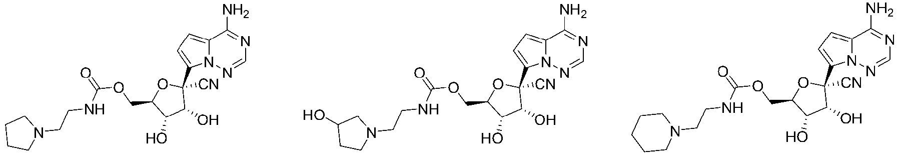

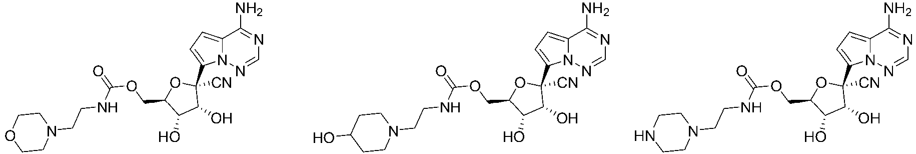

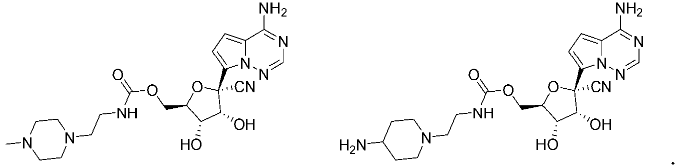

2. Composition pharmaceutique contenant des excipients ou des supports pharmaceutiquement acceptables et le composé ou le sel pharmaceutiquement acceptable selon la revendication 1 en tant que principe actif, dans laquelle le principe actif comprend chaque isomère optique, chaque forme cristalline, hydrate ou solvate de celui-ci.

3. Composé ou sel pharmaceutiquement acceptable de celui-ci selon la revendication 1 pour une utilisation dans le traitement de maladies causées par une infection par le coronavirus félin.

4. Composition pharmaceutique selon la revendication 2 pour une utilisation dans le traitement de maladies causées par une infection par le coronavirus félin.

5. Composé ou sel pharmaceutiquement acceptable de celui-ci pour une utilisation selon la revendication 3, dans lequel la maladie causée par l'infection par le coronavirus félin est la péritonite infectieuse féline.

6. Composition pharmaceutique pour une utilisation selon la revendication 4, dans laquelle la maladie causée par l'infection par le coronavirus félin est la péritonite infectieuse féline.\
